(19) Europäisches Patentamt
European Patent Office
Office européen des brevets

(11) EP 0 478 729 B1

(12) EUROPEAN PATENT SPECIFICATION

(45) Date of publication and mention of the grant of the patent: **21.08.1996 Bulletin 1996/34**

(21) Application number: **91906948.4**

(22) Date of filing: **12.03.1991**

(51) Int Cl.[6]: **C07K 5/06**, C07D 241/08

(86) International application number: **PCT/US91/01646**

(87) International publication number: **WO 91/14378 (03.10.1991 Gazette 1991/23)**

(54) **PROCESS FOR MANUFACTURING ASPARTAME FROM A DIKETOPIPERAZINE AND NOVEL INTERMEDIATES AND DERIVATIVES THEREFOR**

PROZESS ZUR DARSTELLUNG VON ASPARTAM AUS EINEM DIKETOPIPERAZIN SOWIE NEUE ZWISCHENPRODUKTE UND DERIVATE DAFÜR

PROCEDE DE FABRICATION D'ASPARTAME A PARTIR D'UNE DICETOPIPERAZINE ET NOUVEAUX INTERMEDIAIRES ET DERIVES PREVUS A CET EFFET

(84) Designated Contracting States:
**AT BE CH DE DK ES FR GB GR IT LI LU NL SE**

(30) Priority: **15.03.1990 US 498243**
**31.10.1990 US 606656**
**26.02.1991 US 658784**
**08.03.1991 US 664806**

(43) Date of publication of application: **08.04.1992 Bulletin 1992/15**

(73) Proprietor: **THE NUTRASWEET COMPANY**
**Deerfield, IL 60015 (US)**

(72) Inventors:
- **HILL, John, B.**
  **Woodstock, IL 60098 (US)**
- **HO, Tse-Lok**
  **Glenview, IL 60025 (US)**
- **JOHNSON, Mark, R.**
  **Grayslake, IL 60030 (US)**
- **KLIX, Russell**
  **Buffalo Grove, IL 60089 (US)**
- **WEBBER, Gayle**
  **Evanston, IL 60201 (US)**
- **ERICKSON, Robert, A.**
  **Des Plaines, IL 60016 (US)**

(74) Representative: **Blum, Rudolf Emil Ernst et al**
**c/o E. Blum & Co**
**Patentanwälte**
**Vorderberg 11**
**8044 Zürich (CH)**

(56) References cited:
**US-A- 3 775 332** **US-A- 4 634 790**
**US-A- 4 638 081** **US-A- 4 673 744**
**US-A- 4 677 220** **US-A- 4 684 745**
**US-A- 4 760 164** **US-A- 4 780 561**
**US-A- 4 992 552**

- **RESEARCH DISCLOSURE. vol. 281, 1 September 1987, NEW YORK , NY, USA page 552 J C HUBBS 'synthesis of n-acetyl-l-alpha-aspartyl-l-phenylalanine methyl ester'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

1. Field of the Invention

This invention relates to a process for manufacturing aspartame (α-L-aspartyl-L-phenylalanine methyl ester ("α-APM")) from a diketopiperazine ("DKP") as well as novel intermediates produced in the process. This invention particularly relates to a process for manufacturing α-APM by use of 1-acyl-5(S)-benzyl-3,6-dioxopiperazine-2(S)-acetic acid ("1-acyl AP-DKP") or methyl 1-acyl-5(S)-benzyl-3,6-dioxopiperazine-2(S)-acetate ("1-acyl MAP-DKP"). By utilizing the present process, α-APM is manufactured without the use of L-phenylalanine or its methyl ester as a starting material.

2. Description of the Prior Art

α-APM is a compound used as a sweetening agent. It is typically manufactured by processes which employ L-phenylalanine or L-phenylalanine methyl ester as one of the starting coupling materials, which are major cost factors in the manufacturing process.

One attempt to produce α-APM without using L-phenylalanine methyl ester is found in U.S. Patent No. 4780561 to Mita et al. The patent teaches a process by which 5-benzyl-3,6-dioxo-2-piperazine acetic acid ("AP-DKP") or its methyl ester is contacted with hydrochloric acid to produce α-L-aspartyl-L-phenylalanine methyl ester hydrochloride, which is neutralized to produce α-APM. The 5-benzyl-3,6-dioxo-2-piperazine acetic acid is prepared by the deformylation and diesterification of N-formyl-α-L-aspartyl-L-phenylalanine in methanol in the presence of an acid to form α-L-aspartyl-L-phenylalanine dimethyl ester. The dimethyl ester is then treated under neutral or slightly alkaline conditions to form methyl 5-benzyl-3,6-dioxo-2-piperazine acetate ("MAP-DKP"). The HAP-DKP is then treated with an aqueous alkaline solution to form the AP-DKP. This process still requires L-phenylalanine as a raw material, which adds greatly to the amount of the process costs.

U.S. Patent No. 4634790 to Shinohara et al discloses a process for producing α-APB or its hydrohalide by subjecting 3-benzyl-6-carboxymethyl-2,5-diketopiperazine ("α-AP-DKP") to partial hydrolysis with a strong acid in a solvent mixture of methanol and water. The α-AP-DKP is typically obtained as a by-product formed during commercial production of α-APM.

Japan Published Application No. 01-100161 discloses 5-benzyl-3,6-dioxo-2-piperazine acetic acid (AP-DKP) and its derivatives. The compounds are prepared by contacting $C_6H_5CH_2CH(NH_2)CONHCH(CO_2R_1)CH_2CO_2R_2$ ($R_1$=$C_1$-$C_4$ alkyl, $R_2$=H,$C_1$-$C_4$ alkyl) with aqueous solutions or $H_2O$-organic solvent mixtures whose pH is greater than or equal to 4.5. APM-HCl is produced by heating AP-DKP in methyl alcohol containing HCl.

Hubbs, in Research Disclosure 28136, published September 1987, discloses the synthesis of N-acetyl-α-L-aspartyl-L-phenylalanine methyl ester without using L-phenylalanine or its methyl ester as a starting material. The compound is converted to α-L-aspartyl-L-phenylalanine, which can then be converted to aspartame.

U.S. Patent No. 4897507 to Takahashi et al discloses a method for producing α-L-aspartyl-L-phenylalanine dimethyl ester by reacting 3-benzyl-6-carboxymethyl-2,5-diketopiperazine or its methyl ester in a methanolic solvent substantially free of water. The produced dimethyl ester is converted to α-APM (hydrochloride salt) by reaction in an acidic aqueous methanolic solution.

It is desirable to produce α-APM from diketopiperazines without requiring the use of L-phenylalanine or L-phenylalanine methyl ester as raw materials. The present invention teaches such a process.

Summary of the Invention

This invention provides novel processes for the production of 1-acyl AP-DKP and 1-acyl MAP-DKP, which in turn can easily be converted to α-APM. These compositions are produced without using L-phenylalanine or its methyl ester in the synthesis. In carrying out the processes of the present invention, the following novel intermediate compounds and classes of intermediate compounds are formed:

1,4-diacyl-3,6-dioxopiperazine-2(S)-acetic acid

alkyl 1,4-diacyl-3,6-dioxopiperazine-2(S)-acetate

1-acyl-5-benzylidene-3,6-dioxopiperazine-2(S)-acetic acid

alkyl 1-acyl-5-benzylidene-3,6-dioxopiperazine-2(S)-acetate

5-benzylidene-3,6-dioxopiperazine-2(S)-acetic acid

alkyl 5-benzylidene-3,6-·dioxopiperazine-2(S)-acetate

alkyl 1-acyl-5(S)-benzyl-3,6-dioxopiperazine-2(S)-acetate.

1-acyl-5-(acyloxybenzyl)-3,6-dioxopiperazine-2(S)-acetic acid

alkyl 1-acyl-5-(acyloxybenzyl)-3,6-dioxopiperazine-2(S)-acetate

N-acyl-α-L-aspartyl-L-phenylalanine dialkyl ester

In accordance with one embodiment, the present invention provides a process for producing aspartame (α-APM) comprising the steps in the order of :

(a) adding one or more acylating agents to 3,6-dioxopiperazine-2(S)-acetic acid to form a 1,4-diacyl-3,6-dioxopiperazine-2(S)-acetic acid compound;

(b) adding benzaldehyde to said 1,4-diacyl-3,6-dioxopiperazine-2(S)-acetic acid compound in the presence of a base to form a 1-acyl-5-benzylidene-3,6-dioxopiperazine-2(S)-acetic acid compound;

(c) hydrogenating said 1-acyl-5-benzylidene-3,6-dioxopiperazine-2(S)-acetic acid compound to produce a 1-acyl-5(S)-benzyl-3,6-dioxopiperazine-2(S)-acetic acid compound; and

(d) partially hydrolyzing said 1-acyl-5(S)-benzyl-3,6-dioxopiperazine-2(S)-acetic acid compound to produce α-APM or a salt thereof.

In the above-described products and processes, the acyl groups are of the formula RC(=O)- wherein R represents hydrogen or a straight chain, branched chain, cyclic or aromatic organic group containing between 1 and 8 carbon atoms. With respect to diacyl compounds, each acyl group may be the same or different. In preferred embodiments, the acyl groups comprise acetyl groups.

The 3,6-dioxopiperazine-2(S)-acetic acid starting compound in the above synthesis can be prepared in a three-step reaction wherein:

i) L-aspartic acid is reacted with chloroacetyl-chloride to form chloroacetylaspartic acid;

ii) chloroacetylaspartic acid is reacted with ammonia to form the dipeptide, Gly·Asp; and

iii) Gly·Asp is cyclized (by heating) to form the desired diketopiperazine, viz., 3,6-dioxopiperazine-2(S)-acetic acid (cyclic Gly·Asp).

Another embodiment of the invention provides a second process for producing aspartame (α-APM) comprising the steps in the order of:

(a) adding one or more acylating agents to an alkyl 3,6-dioxopiperazine-2(S)-acetate compound to form an alkyl 1,4-diacyl-3,6-dioxopiperazine-2(S)-acetate compound;

(b) adding benzaldehyde to said alkyl 1,4-diacyl-3,6-dioxopiperazine-2(S)-acetate compound in the presence of a base to form an alkyl 1-acyl-5-benzylidene-3,6-dioxopiperazine-2(S)-acetate compound;

(c) hydrogenating said alkyl 1-acyl-5-benzylidene-3,6-dioxopiperazine-2(S)-acetate compound to form an alkyl 1-acyl-5(S)-benzyl-3,6-dioxopiperazine-2(S)-acetate compound;

(d) adding an alcohol under neutral or basic conditions to said alkyl 1-acyl-5(S)-benzyl-3,6-dioxopiperazine-2(S)-acetate compound to produce a mixture including N-acyl-α-L-aspartyl-L-phenylalanine dialkyl ester; and

(e) adding BCl and $CH_3OH$ to said mixture produced in step (d);

vherein acyl and diacyl are as defined above; and wherein alkyl comprises a straight chain, or branched chain alkyl group containing between 1 and 7 carbon atoms.

In still another embodiment, instead of conducting step (d) of the above process under neutral or basic conditions to produce a mixture including N-acyl-alpha-L-aspartyl-L-phenylalanine dialkyl ester, the direct conversion of the alkyl 1-acyl-5(S)-benzyl-3,6-dioxopiperazine-2(S)-acetate compound to alpha-APM or a salt thereof step can be accomplished under acidic conditions.

In particularly preferred embodiments, the mother liquor remaining after the isolation of α-APM in either of the above processes is converted to 5(S)-benzyl-3,6-dioxopiperazine-2(S)-acetic acid (AP-DKP) or methyl 5(S)-benzyl-3,6-dioxopiperazine-2(S)-acetate ("MAP-DKP"), which in turn is then partially hydrolyzed to produce additional α-APM. This improves the overall yield of the process.

Accordingly, it is an object of the present invention to provide novel processes for producing α-APM and intermediates necessary to produce α-APM.

It is a further object of the present invention to provide novel compounds which are particularly useful as intermediates for the production of α-APM.

These and other objects will be readily apparent to one skilled in the art as reference is made to the detailed description of the preferred embodiment.

## Detailed Description of the Preferred Embodiment

When describing the preferred embodiment, certain terminology will be used for the sake of clarity. The use of such terminology encompasses the recited embodiment, as well as all technical equivalents which operate in a similar manner for a similar purpose to achieve a similar result.

An overall reaction scheme for forming α-APM by utilizing a preferred process of the present invention is shown in the following diagram.

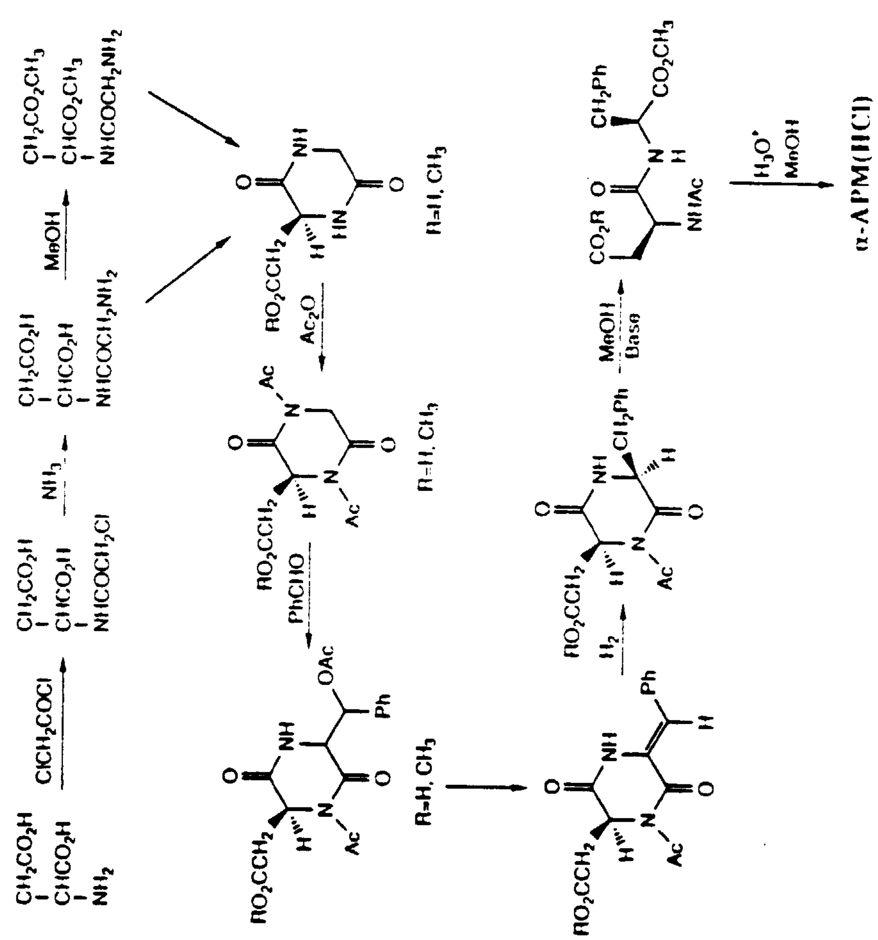


Referring to the reaction diagram, the 1-acyl AP-DKP compounds can be prepared in accordance with the following reaction scheme: L-Aspartic Acid is first reacted with chloroacetylchloride in a solvent such as ethyl acetate to form chloroacetylaspartic acid. Other solvents including methyl acetate, propyl acetate and the like may be selected. The

chloroacetylaspartic acid is then reacted with ammonia, typically aqueous ammonia, to produce Gly·Asp, a dipeptide having two carboxyl groups and a single primary amine group. The reaction with ammonia may take place at room temperature, but this requires a relatively long period of time for the reaction to occur; i.e. 12 hours or more. To reduce the reaction time, the ammonation may take place at higher temperatures. Temperatures may range from about 50 to about 250°C. This in turn, corresponds to a reaction rate of between about three hours and about one second.

The next step in the procedure is to form a second peptide linkage by means of a cyclization reaction. Gly·Asp can itself be cyclized to form 3,6-dioxopiperazine-2(S)-acetic acid (cyclic Gly·Asp). Alternatively, either (or preferably both) of the carboxyl groups in Gly·Asp can be esterified with a lower alkyl alcohol having from one to five carbon atoms (e.g. methyl, ethyl, propyl, iso-butyl and the like) in the presence of an acid (e.g., $HCl, H_2SO_4$) or acid resin before cyclization. Methanol is especially preferred, in which case the acid salt of the following intermediate is formed prior to cyclization:

$$CH_2COOCH_3$$
$$|$$
$$CHCOOCH_3$$
$$|$$
$$NHCOCH_2NH_2$$

Other starting materials may be selected for cyclization into the DKP, including the beta-methyl ester of Gly·Asp. Those skilled in the art will readily appreciate other compounds which may be used as precursors to form the DKP.

Cyclization of Gly·Asp typically occurs by heating the compound. When heating Gly·Asp to form the first DKP (cyclic Gly·Asp), the dipeptide is heated to between about 100 and about 210°C for a time period ranging from about 30 minutes to about 5 hours. It is particularly preferred, although not required, to perform the heating step in the presence of a lover alkyl carboxylic acid, such as acetic, propionic or pivalic acid. By heating in a lower alkyl carboxylic acid, the temperature of heating may be reduced to between about 100 and about 130 °C and the heating time may be reduced to between about 1 and about 3 hours. Alternatively, other solvents, such as hydrocarbons, dimethylformamide or dimethyl sulfoxide may be utilized as the heating medium.

Cyclization of the alkyl ester of Gly·Asp occurs by neutralization of the acid salt with a base. The base can take the form of an organic base, inorganic base, or basic resin.

One or more acylating agents is then added to cyclic Gly·Asp or its alkyl ester to form the novel compounds 1,4-diacyl-3,6-dioxopiperazine-2(S)-acetic acid and alkyl 1,4-diacyl-3,6-dioxopiperazine-2(S)-acetate. The term "acyl" represents a group of the formula RC(=O)- wherein R represents hydrogen, a straight chain, branched chain, cyclic or aromatic group containing between 1 and 8 carbon atoms and the term "diacyl" represents two acyl groups which may be the same or different. Examples of acyl groups include formyl, acetyl, propionyl, benzoyl, and the like. Particularly preferred acyl groups comprise formyl and acetyl groups. The term alkyl represents a straight chain, or branched chain alkyl group having between 1 and 7 carbon atoms.

At least two equivalents of acyl groups must be added per equivalent of DKP. In a preferred embodiment, the acylating agent comprises acetic anhydride such that a 1,4-diacetyl substituted compound is produced or formyl acetic anhydride (mixed anhydride) such that a 1,4-diformyl substituted compound is produced. Other acylating agents well known in the art such as acetyl chloride and ketene may be used in place of acetic anhydride or formyl acetic anhydride. Catalysts such as dimethylaminopyridine or sodium acetate may also be added. The addition of the acylating reagent typically takes place at elevated temperatures, ranging between about 25°C and about 130°C. The acylation reaction may take place in any inert solvent such as ethyl acetate, toluene, isopropyl acetate, xylene, acetic acid and the like. Alternatively, an excess of acylating agent may be provided to additionally function as a reaction solvent. The use of an excess amount of acetic anhydride is one such example.

In the case of cyclic Gly·Asp, it may be desirable to replace the carboxylic hydrogen atom of the carboxymethyl group attached to the number 2 carbon atom with a protective group prior to the acylation reaction to prevent interference from the hydrogen atom during the acylation reaction. Examples of protective groups include trimethylsilyl, tert-butyl and tert-butyl-dimethylsilyl. Other protective groups will be appreciated by those skilled in the art.

In the next step of the reaction sequence, benzaldehyde is added to the 1,4-diacyl-3,6-dioxopiperazine intermediate (free acid or alkyl ester) in the presence of a base to form either of the following classes of novel compounds: 1-acyl-5-benzylidene-3,6-dioxopiperazine-2(S)-acetic acids, or alkyl 1-acyl-5-benzylidene-3,6-dioxopiperazine-2-(S)-acetates (1-acetyl or 1-formyl compounds are especially preferred). Alkoxide compounds are particularly suitable for use as bases in the above reaction. Specific examples include sodium tert-butoxide, potassium tert-butoxide and sodium tert-amyloxide. The benzaldehyde addition takes place in the presence of an inert solvent such as tetrahydrofuran or

tert-butyl alcohol, and preferably at temperatures ranging between about -40°C and about 100°C.

When producing alkyl 1-acyl-5-benzylidene-3,6-dioxopiperazine-2-(S)-acetates, an intermediate, alkyl l-acyl-5-(acyloxybenzyl)-3,6-dioxopiperazine-2(S)-acetate is initially produced (acyl preferably is acetyl or formyl). In practice, this intermediate need not be specifically isolated, but rather, the alkyl 1,4-diacyl-3,6-dioxopiperazine-2(S)-acetate is directly converted to alkyl 1-acyl-5-benzylidene-3,6-dioxopiperazine-2(S)-acetate through an alkyl 1-acyl-5-(acyloxybenzyl)-3,6-dioxopiperazine-2(S)-acetate intermediate. While it has not been specifically isolated, the preferred formyl compound is believed to be methyl N-formyl-5-(formyloxybenzyl)-3,6-dioxopiperazine-2(S)-acetate. When isolated, conversion of the 5-(acyloxybenzyl) compound is accomplished by adding a base compound to the intermediate followed by heating the mixture and thereafter quenching the compound with an acid. The addition typically takes place in an inert solvent such as cyclohexane. While not specifically tested, it is also believed that a 5-(acyloxybenzyl) intermediate is produced during the benzylidene addition to the free acid, i.e., that 1-acyl-5-(acyloxybenzyl)-3,6-dioxopiperazine-2(S)-acetic acid is produced.

In the case of the free acid intermediates, if the carboxylic hydrogen atom of the carboxymethyl group substituted on the number 2 carbon atom has been replaced with a protective group other than lower alkyl, the protective group should be replaced with either a hydrogen atom or an alkyl group (preferably methyl) to return the DKP to the acetic acid or alkyl acetate form. The replacement of the protective group occurs after the benzaldehyde addition.

In alternative embodiments, the 1-acyl-5-benzylidene-3,6-dioxopiperazine-2(S)-acetic acid or alkyl 1-acyl-5-benzylidene-3,6-dioxopiperazine-2(S)-acetate compounds are deacylated by the addition of methanol or other suitable solvents such as water. Upon complete reaction the novel compounds 5-benzylidene-3,6-dioxopiperazine-2(S)-acetic acid and alkyl 5-benzylidene-3,6-dioxopiperazine-2(S)-acetate are produced.

The intermediates formed as a result of the foregoing reactions are partially unsaturated. These intermediates can be hydrogenated to form the desired 1-acyl AP-DKP, 1-acyl alkyl-AP-DKP, AP-DKP and alkyl-AP-DKP compounds, systematically named, 1-acyl-5(S)-benzyl-3,6-dioxopiperazine-2(S)-acetic acid, alkyl 1-acyl-5(S)-benzyl-3,6-dioxopiperazine-2(S)-acetate, 5(S)-benzyl-3,6-dioxopiperazine-2(S)-acetic acid and alkyl 5(S)-benzyl-3,6-dioxopiperazine-2 (S)-acetate respectively. The conversion can be accomplished by using hydrogen gas or a hydrogen liberating material in the presence of a hydrogenation catalyst. Examples of catalysts which may be used include Pd on a support such as C, $BaCO_3$, $BaSO_4$, alumina, $CaCO_3$ and the like, Pt on a support, Ni catalysts, Cu catalysts, Rh catalysts and soluble metal catalysts. The hydrogenation reaction typically occurs at temperatures ranging from about -20°C to about 150°C. The hydrogenation reaction can take place in a large number of inert solvents which are typically used in hydrogenation reactions, wherein as known in the art solvent selection is largely determined by the catalyst selected. In preferred embodiments, 1-formyl AP-DKP, 1-formyl alkyl AP-DKP, 1-acetyl AP-DKP or 1-acetyl alkyl AP-DKP is produced.

The AP-DKP, alkyl-AP-DKP, 1-acyl AP-DKP and 1-acyl alkyl-AP-DKP compounds can all easily be converted to α-APM. To convert any of the above listed DKPs to the acid salt of α-APM, all that is required is that the DKP be partially hydrolyzed. This typically involves adding methanol and acid, typically HCl to the DKP at elevated temperatures followed by mixing for a period of time sufficient to enable the α-APM hydrochloride salt to form. Once formed, the hydrochloride salt is converted to α-APM by conventional methods such as neutralization and the α-APM is thereafter isolated from the remainder of the mother liquor. The partial hydrolysis of DKPs to form α-APM is described in greater detail in previously cited U.S. Patent Nos. 4,634,790 and 4,897,507.

In an alternative embodiment, instead of using acidic hydrolysis of the DKP compounds to yield α-APM, the hydrolysis may be done under basic or neutral conditions. In this embodiment, a straight chain, or branched chain alcohol containing between 1 and 7 carbon atoms, preferably methanol, is added to an alkyl l-acyl-5(S)-benzyl-3,6-dioxopiperazine-2(S)-acetate compound under basic or neutral conditions to produce a N-acyl-α-L-aspartyl-L-phenylalanine dialkyl ester compound, preferably N-acetyl-α-L-aspartyl-L-phenylalanine dimethyl ester or N-formyl-α-L-aspartyl-L-phenylalanine dimethyl ester. The "alkyl" groups of these "dialkyl" compounds may be the same or different and comprise straight or branched chain groups containing between 1 and 7 carbon atoms. These N-acyl compounds are considered to be novel.

In practice, the pH of this hydrolysis reaction is maintained between about 6.5 and about 14, preferably between about 7.0 and about 8.5 by adding a base with the alcohol at temperatures ranging from about -20°C to about 120°C, preferably from about 20°C to about 60°C for a time period ranging from about 1 minute to about 10 days, preferably from about 15 minutes to about 24 hours. Preferred bases which may be added include: sodium acetate, sodium carbonate, sodium bicarbonate, sodium hydroxide, lithium hydroxide, ammonia, organic amine bases and potassium bases. For example, the addition of 0.25 equivalents of sodium acetate per equivalent of alkyl 1-acyl-5(S)-benzyl-3,6-dioxopiperazine-2(S)-acetate yields satisfactory results.

In practice, in addition to the formation of the N-acyl-α-L-aspartyl-L-phenylalanine dialkyl ester compound, an amount of methyl 5(S)-benzyl-3,6-dioxopiperazine-2(S)-acetate (MDKP) is also formed. In practice, the ratio of the two compounds formed ranges from 50:50 to 67:33, with a ratio of 65:35 having actually been produced. The high relative yield of the N-acyl-α-L-aspartyl-L-phenylalanine dialkyl ester compound is considered surprising as cleavage of the imide functional group can occur at either of the carbonyls, resulting in either deacylation to MDKP or ring-opening to

N-acyl-α-L-aspartyl-L-phenylalanine dialkyl ester. Under acidic conditions, deacylation is the exclusive reaction. The observed selectivity under basic or neutral conditions is surprising, as steric arguments would suggest deacylation would be preferred. Arguments based on electronic effects would predict only modest differences between the two carbonyls.

Further hydrolysis of these two compounds by utilizing methanol, an acid, preferably HCl, and optionally water yields a mixture of methyl α-L-aspartyl-L-phenylalanine methyl ester (MAPM (desired)) and methyl α-L-phenylalanine-L-aspartyl methyl ester (PAMM (not desired)) in a high ratio of desired to undesired product. For example, a final ratio of 90:10 MAPM to PAMM can be produced using the above process steps. The MAPM can easily be converted to α-APB using known techniques, namely adding additional methanol, $H_2O$ and acid to the MAPM.

The above processes are capable of yielding up to about 80% of α-APB based upon the amount of initial DKP synthesized. After isolation of α-APM from the mother liquor, the remainder of the mother liquor contains quantities of Asp, Phe, Asp·Phe, Phe·Asp and their methyl esters (as well as an amount of nonisolated APM(HCl)). To convert these materials to AP-DKP or MAP-DKP (methyl ester of AP-DKP) so that additional α+APM can be synthesized, the pH of the mother liquor is adjusted, typically to between about 1.5 and 6.0, and the liquor is heated to between about 40°C and about 110°C until the dipeptides and their methyl esters cyclize. The DKPs are isolated by cooling and filtering.

To improve the conversion of the dipeptides in the mother liquor to AP-DKP or MAP-DKP, the cyclization reaction may take place in the presence of an optional co-solvent. One suitable co-solvent is acetic acid. Other co-solvents may include formic acid, propanoic acid, other carboxylic acids and other inert solvents. When a co-solvent is employed, to isolate the DKP the co-solvent is optionally stripped from the mixture, and the liquor is cooled and filtered.

Once isolated, the AP-DKP and/or MAP-DKP is converted to α-APM by partial hydrolysis and neutralization as described above. The re-conversion of the mother liquor can be repeated as often as desired by using this procedure. As a result of the additional α-APM obtained from the mother liquor, overall yields of α-APM as high as 90% can be achieved.

The invention is further described by the following non-limiting examples.

EXAMPLE 1 -- Chloroacetylation of L-Asp by direct acylation

53.24 grams of L-Asp, 32 ml of chloroacetyl chloride and 400 ml of ethyl acetate were heated with stirring to reflux in a 1 liter 3 neck round bottom flask equipped with a condenser. The reaction mixture was refluxed for 24 hrs, forming a slurry. The slurry was cooled to 25 °C and filtered, and the filter cake was washed with 50 ml of ethyl acetate. The cake was dried and the ethyl acetate was removed by vacuum distillation at 40-50 °C. 85 ml of $H_2O$ were added to the solid cake and the solution was stirred and allowed to stand overnight. The water was removed from the solution to liberate a semi-crystalline residue. The residue was stirred with 300 ml of ethyl ether, and was filtered, rinsed and dried to liberate the final product. Air drying afforded 66.95 grams of chloroacetyl aspartic acid, or 80% of theoretical yield.

EXAMPLE 2 -- Formation of Gly·Asp from chloroacetylaspartic acid

5.0 grams of chloroacetylaspartic acid were stirred in 28% aqueous $NH_4OH$ as a clear solution at room temperature for 24 hours. After this time, the $NH_3$ and $H_2O$ were removed by heating under vacuum to 50 °C. 20 ml of water were added to provide a clear solution, followed by the addition of 20 ml of acetic acid. The solution was diluted while stirring with 100 ml methanol to liberate a crystalline precipitate of Gly·Asp. The yield of the air dried product was nearly quantitative based on theoretical yield. The amount of $NH_4^+$ present was 0.12%. HPLC analysis confirmed that Gly·Asp had been formed.

EXAMPLE 2A -- Formation of Gly·Asp using high temperature ammonation step

A slurry of 39.9 g of L-Asp in 300 mL of isopropyl acetate was heated to reflux, and 9.6 mL of chloroacetyl chloride were added. The slurry was refluxed with a nitrogen purge for 4 hours, filtered while hot, and the filtrate was stripped under reduced pressure to yield 19.75g of a white solid. 10g of this material was dissolved in 30mL of water, stirred for 2 hours, and added to 170mL of 28% aqueous ammonia ($NH_4OH$). This solution was heated in a pressure vessel to 105°C for 10 minutes, was immediately cooled to 75°C, and then further cooled with venting to 45°C. The solution was concentrated under reduced pressure, and water was added to produce a total weight of 30g. The pH of the solution was adjusted with 12 N HCl to 3.0, and the solution was added to refluxing methanol over a 30 minute period. After cooling overnight, the product was collected by filtration and dried. The yield of Gly·Asp based on chloroacetyl chloride was 57%.

EXAMPLE 2B -- Formation of Gly·Asp from chloroacetylaspartic acid

A slurry of 213 g of L-Aspartic Acid in 800 mL of isopropyl acetate was heated to reflux, and treated with 63 mL of chloroacetyl chloride. The slurry was refluxed for 3.5 hours, filtered, and the filtrate was stripped under reduced pressure. The residue was treated with 200 mL of isopropyl acetate and 84 mL water, stirred to dissolution, and the aqueous layer was removed. The organic layer was extracted 4 times with 42 mL water per extraction, and the combined aqueous layers were treated with 2.00 1 of 28 % aqueous ammonia. After standing overnight, the solution was stripped under reduced pressure, and treated with 12 N HCl to a pH of 3.45. Approximately half of this material was used for crystallization. The aqueous concentrate was added dropwise over 30 minutes to 1.00 1 of methanol, stirred for 65 hours, chilled to 10°C, and the product was collected by filtration. The yield of Gly·Asp was 60 %, based on chloroacetyl chloride.

EXAMPLE 3 -- Cyclization of Gly·Asp to cyclic Gly·Asp

Three different methods were used to cyclize Gly·Asp.

Method (a)

1.00 gram of Gly·Asp and 20 ml of propionic acid were stirred at reflux (128 °C) for 2 hr, whereupon the solution became clear. The solvent was removed to yield c-Gly·Asp. The yield was 83%.

Method (b)

0.10 gram of Gly·Asp was heated in a tube at 150-160°C under a vacuum of 133 Pa (1 mm Hg for) 30 minutes. The yield of c-Gly·Asp was 57%.

Method (c)

1.00 gram of Gly·Asp and 10 ml of dimethyl sulfoxide were heated to 120 °C for 3 hours, during which the initially colorless solution turned orange. The solvent was removed under a stream of $N_2$ and pumped at 13.3 Pa (0.1 mm Hg) vacuum overnight. The product was semi-crystalline and was recovered in a yield of 90%.

EXAMPLE 4 -- Formation of dimethyl ester of Gly·Asp

A solution of 0.5 grams of Gly·Asp in 10 ml of 10% HCl in methanol was stirred at ambient temperature for 16 hours. The solvent was stripped under reduced pressure to yield the hydrochloride salt of Gly·Asp dimethyl ester in essentially quantitative amounts.

EXAMPLE 5 -- Formation of methyl 3,6-dioxpierazine-2(S)-acetate

A solution of crude Gly·Asp dimethyl ester was prepared from 0.5 g of Gly·Asp. The crude product was stripped under reduced pressure, redissolved in 10 ml of methanol, and treated with a strong base resin to a pH of 8.4 The solution was filtered, allowed to stand at room temperature for 24 hours, and stripped to yield 0.31 g of methyl 3,6-dioxopiperazine-2(S)-acetate.

EXAMPLE 5A -- Formation of methyl 3,6-dioxopiperazine-2(S)-acetate

A slurry of 75mL Dowex MSC-1 resin (MeOH washed) in 200mL methanol was treated with 9.7g Gly·Asp, and heated to reflux. After 3 hours at reflux, the temperature was reduced to 60°C, and the slurry was treated with 28% aqueous ammonia ($NH_4OH$) to a pH of 8.5. After an additional 1.5 hours at 60°C the resin was filtered off, washed, and the filtrate was stripped under reduced pressure to yield methyl 3,6-dioxopiperazine-2(S)-acetate. The yield was 98%.

EXAMPLE 5B -- Formation of methyl 3,6-dioxopiperazine-2(S)-acetate

A solution of 5.0 grams of Gly·Asp and 3.1 grams of $H_2SO_4$ in 100ml of methanol was stirred at 60°C for 5.5 hours. The solution was cooled to ambient temperature and passed down a 75ml weak base resin column. The column was washed with methanol and the wash liquor was combined with the solution. The pH of the solution was adjusted to 8.5

by adding methanolic ammonia. After heating at 60°C for 5 hours, the solution was cooled to ambient temperature and stripped under reduced pressure to yield a white solid containing 4.41 grams of methyl 3,6-dioxopiperazine-2(S)-acetate. The yield was 90%.

EXAMPLE 6 -- Formation of 1,4-diacetyl-3,6-dioxopiperazine-2(S)-acetic acid

172 mg (1 mmol) of cyclic Gly·Asp was slurried in 20 ml of ethyl acetate. 0.566 ml (6 mmol) of acetic anhydride and 4 mg of dimethylaminopyridine (DMAP) as a catalyst were added to the slurry and the mixture was heated to reflux for 25 hours and cooled to room temperature. The mixture was washed with water, dried with $MgSO_4$ and concentrated under vacuum to yield 121 mg of 1,4-diacetyl-3,6-dioxopiperazine-2(S)-acetic acid. The yield was 47%.

EXAMPLE 7 -- Formation of methyl 1,4-diacetyl-3,6-dioxopiperazine-2(S)-acetate

A slurry of 372 mg (2 mmol) of methyl 3,6-dioxopiperazine-2(S)-acetate in 10 ml of acetic anhydride was heated to above 50°C. The reaction mixture was stirred at this temperature until the reaction was completed and the product was then concentrated to afford an essentially quantitative yield of methyl 1,4-diacetyl-3,6-dioxopiperazine-2(S)-acetate.

EXAMPLE 7A -- Formation of methyl 1,4-diacetyl-3,6-dioxopiperazine-2(S)-acetate

A solution of 100g methyl 3,6-dioxopiperazine-2(S)-acetate in 1300mL of acetic anhydride was heated at 100°C for 7 hours, and then stripped under reduced pressure. The residue was dissolved in 1000mL of butyl acetate, filtered, washed with a pH 6.3 buffer, dried over sodium sulfate, and stripped again under reduced pressure to yield methyl 1,4-diacetyl-3,6-dioxopiperazine-2(S)-acetate as an oil. The yield was 96%.

EXAMPLE 8 -- Formation of methyl 1-acetyl-5-benzlidene-3,6-dioxopiperazine-2(S)-acetate

To a solution of 2.15 g of methyl-1,4-diacetyl-3,6-dioxopiperazine-2(S) acetate and 0.845 g of benzaldehyde in 20 mL of THF at 5 deg C was added 0.964 g of sodium tert.-pentyloxide The slurry was stirred at 5 deg C for 15 minutes and then allowed to warm to room temperature and stirred an additional 7 hours. The mixture was quenched with acetic acid and poured in water and extracted with methylene chloride. The organic layer was dryed and concentrated to afford 80 % of methyl 1-acetyl-5-benzylidene-3,6-dioxopiperazine-2(S) acetate.

EXAMPLE 8A -- Formation of methyl 1-acetyl-5-benzylidene-3,6-dioxopiperazine-2(S)-acetate

To a solution of 2.90g (10 mmol) of methyl 1,4-diacetyl-3,6-dioxopiperazine-2(S)-acetate in 15mL of butyl acetate was added 1.06g (10 mmol) of benzaldehyde. The reaction mixture was cooled to -20°C and 1.05g (11 mmol) of sodium tert-butoxide dissolved in 10mL of cyclohexane was added at -20°C. After addition of the base, the reaction mixture was stirred at -20°C for 1h and .630mL (11 mmol) of acetic acid was then added. The reaction mixture was then heated at 70°C for 10h, cooled to room temperature and washed with water. The organic layer was dried and concentrated to afford 90% of methyl 1-acetyl-5-benzylidene-3,6-dioxopiperazine-2(S)-acetate.

EXAMPLE 8B -- Formation of methyl 1-acetyl-5-(acetoxbenzyl)-3,6-dioxopiperazine-2(S)-acetate

To a solution of 48.64g (.180 mol) of methyl 1,4-diacetyl-3,6-dioxopiperazine-2(S)-acetate in 250mL of butyl acetate was added 19.10g (.180 mol) of benzaldehyde. The reaction mixture was cooled to -20°C and 19.03g (.198 mol) of sodium tert-butoxide dissolved in 200mL of cyclohexane was added at 7°C. After addition of the base, the reaction mixture was stirred an additional 1 minute at -20°C and then quenched with 11.3mL (.198 mol) of acetic acid. The reaction mixture was then warmed to room temperature and concentrated to afford 56% of crude methyl 1-acetyl-5-(acetoxybenzyl)-3,6-dioxopiperazine-2(S)-acetate. Silica gel chromatography afforded pure material as a glassy solid.

EXAMPLE 8C -- Formation of methyl 1-acetyl-5-benzylidene-3,6-dioxopiperazine-2(S)-acetate from methyl 1-acetyl-5-(acetoxybenzyl)-3,6-dioxopiperazine-2(S)-acetate

To a solution of 1.50g (4 mmol) of methyl 1-acetyl-5-(acetoxybenzyl)-3,6-dioxopiperazine-2(S)-acetate in a mixture of 5.5mL of butyl acetate and 4.5mL of cyclohexane was added 0.328g (4 mmol) of sodium acetate. The mixture was heated at 70°C for 24 hours. The crude reaction mixture, after cooling to room temperature and removal of solvents, afforded 74.5% of methyl 1-acetyl-5-benzylidine-3,6-dioxopiperazine-2(S)-acetate.

EXAMPLE 9 -- Formation of methyl 1-acetyl-5(S)-benzyl-3,6-dioxopiperazine-2(S)-acetate

To a solution of 3.16 g of methyl 1-acetyl-5-benzylidene-3,6-dioxopiperazine-2(S) acetate in 95 mL of methanol was added 1.2 g of 4 % Pd/C, 50 % wet with water. The reaction mixture was placed under 50 psi of hydrogen for 1 hour. The catalyst was filtered off using a celite plug and the resulting filtrate concentrated to afford 85 % of methyl 1-acetyl-5(S)-benzyl-3,6-dioxopiperazine-2(S) acetate.

For the formation of 5-benzylidene-3,6-dioxopiperazine-2(S) acetic acid a solution of 1-acetyl-5-benzylidene-3,6-dioxopiperazine-2(S)-acetic acid in aqueous methanol at a pH of 8 is stirred at room temperature until hydrolysis is complete, and the solvent is removed under reduced pressure.

For the formation of methyl 5-benzylidene-3,6-dioxopiperazine-2(S)-acetate a solution of methyl 1-acetyl-5-benzylidene-3,6-dioxopiperazine-2(S)-acetate in aqueous methanol at pH of 8 is stirred at room temperature until hydrolysis is complete, and the solvent is removed under reduced pressure to give the crude desired deacetylated product.

EXAMPLE 10 -- Production of α-APM(HCl) from Methyl 5(S)-benzyl-3,6-dioxopiperazine-2(S)-acetate

A solution of 40 ml of 12 N HC1 and 121 ml of methanol was heated to 60°C. 16 g of MAP-DKP was added over the course of 10 minutes with stirring. The mixture was stirred another 15 minutes at 60°C, at which time the solid had completely dissolved. The solution was stripped on a roto-vap to a residue weighing 33.3 g. After analysis for HCl, methanol and water, the mixture was made up to the following concentrations: methanol--3.0%, ED--13.5%, and water--36.5%. The mixture was shaken at room temperature for seven days and solid APM(HCl) was collected by filtration. The yield of APM(HCl) obtained was 47%.

EXAMPLE 11 -- Conversion of Ac-MDKP to MAPM(HCl)

A solution of 2.16 mg/mL of Ac-MDKP in 10 wt % HCl/methanol was stirred at room temperature for 60 hours, and then analyzed by HPLC for conversion to MAPM.HCl. The yield of MAPM was 86 %, based on the initial charge of Ac-MDKP.

EXAMPLE 12 -- Conversion of mother liquor to AP-DKP or MAP-DKP

(a) A solution of combined mother liquor and wash liquor from conversion of AP-DKP to APM.HCl was used for reconversion to a mixture of DKP/MDKP. This liquor contained 0.577 mM of peptide per gram of solution. The mixture included Asp, Phe, Asp·Phe, Phe·Asp and methyl esters (8 components). A sample of 15.4 g of liquor was treated with 4.32 g of 30% aqueous ammonia and 15 ml water, to give a solution with pH=4.9. This solution was heated at reflux for 6.5 h, stirred overnight at room temperature, and again refluxed for 4 h. The mixture was chilled to 15°C, and the solid collected by filtration. The yield of DKP/MDKP in the cake (solids) was 49%, while the overall yield (amount present in solids and liquid) of DKP/MDKP was 53%.

(b) A sample of 15.39 g of the liquor described in Example 6(a) was treated with 4.5 ml of 30% aqueous ammonia to give a solution of pH = 5.2. This solution was treated with 35 ml of acetic acid, and heated at reflux for 5 hours. The mixture was concentrated under reduced pressure, reslurried in 15 ml water, filtered, and dried. The yield of DKP/MDKP in the cake (solids) was 51%, while the overall yield (amount present in solids and liquid) of DKP/MDKP was 59%.

EXAMPLE 13 -- Formation of N-acetyl-α-L-aspartyl-L-phenylalanine dimethyl ester from Methyl 1-acetyl-5(S)-benzyl-3,6-dioxopiperazine-2(S)-acetate

A solution of 1 mmol of methyl 1-acetyl-5(S)-benzyl-3,6-dioxopiperazine-2(S)-acetate in 10 mL of methanol was adjusted to a pH=7.5 with sodium acetate. The reaction mixture was heated at 60 °C for 4 hours and then cooled to room temperature. The mixture was concentrated under reduced pressure to afford N-acetyl-α-L-aspartyl-L-phenylalanine dimethyl ester and MDKP in a ratio of 64:36.

EXAMPLE 14 -- Conversion of methyl 1-acetyl-5(S)-benzyl-3,6-dioxopiperazine-2(S)-acetate to MAPM

A solution of 2.50 g of methyl 1-acetyl-5(S)-benzyl-3,6-dioxopiperazine-2(S)-acetate in 75 mL of methanol was treated with 0.163 g of sodium acetate and heated to 60 °C. After 1.5 hours the solution was stripped under reduced pressure to yield a white solid containing a 70/30 ratio of N-acetyl-α+L-aspartyl-L-phenylalanine dimethyl ester/ methyl 5(S)-benzyl-3,6-dioxopiperazine-2(S)-acetate. The crude solid was dissolved in 11.5 grams of a 12 % solution of HCl

in methanol, and heated for 6 hours at reflux. An additional 2.3 g of HCl/methanol solution was added and heating continued an additional 10 hours. HPLC analysis showed a mixture of MAPM and PAMM in a ratio of 8.4/1.0.

EXAMPLE 15 -- Formation of methyl 1,4-diformyl-3,6-dioxopiperazine-2(S)-acetate

A solution of 15.0 g methyl 3,6-dioxopiperazine-2(S)-acetate in 50 mL of formyl acetic anhydride (mixed anhydride) was heated at 55 °C for 12 hours, and then stripped under reduced pressure. The residue was redissolved in 50 mL of mixed anhydride and heated again at 55 °C for 12 hours, and then stripped under reduced pressure to yield methyl 1,4-diformyl-3,6-dioxopiperazine-2(S)-acetate as an oil. The yield according to HPLC was 50%.

EXAMPLE 16 -- Formation of methyl 1-formyl-5-benzlidene-3,6-dioxopiperazine-2(S)-acetate

To a solution of 2.42 g of methyl 1,4-diformyl-3,6-dioxpiperazine-2(S)-acetate and 1.06 g of benzaldehyde in 15 mL of isopropyl acetate at -20 °C was added 1.06 g of sodium tert-butoxide dissolved in 10 mL of isopropyl acetate. After addition of the base, the reaction mixture was stirred at -20 °C for 1h and 114 µl of acetic acid was added. The reaction mixture was then heated at 70 °C for 1h, cooled to room temperature and washed with water. The organic layer was dried and concentrated to afford 50% of methyl 1-formyl-5-benzylidene-3,6-dioxopiperazine-2(S)-acetate according to HPLC.

EXAMPLE 17 -- Formation of methyl 1-formyl-5(S)-benzyl-3,6-dioxopiperazine-2(S)-acetate

To a solution of 100 mg of methyl 1-formyl-5-benzylidene-3,6-dioxopiperazine-2(S)-acetate in 10 mL of tetrahydrofuran was added 35mg of a 5% palladium on carbon catalyst. The reaction mixture was placed under 0.345 mPa (50 psi) of hydrogen for 3 hours. The catalyst was filtered off using a celite plug and the resulting filtrate was concentrated to afford 87% of methyl 1-formyl-5(S)-benzyl-3,6-dioxopiperazine-2(S)-acetate according to HPLC.

EXAMPLE 18 -- Formation of N-formyl-alpha-L-aspartyl-L-phenylalanine dimethyl ester from methyl 1-formyl-5(S)-benzyl-3,6-dioxopiperazine-2(S)-acetate

A solution of 5 mg of methyl 1-formyl-5(S)-benzyl-3,6-dioxopiperazine-2(S)-acetate in 0.5 mL of methanol was adjusted to a pH=8.0 with 10% aqueous sodium carbonate and was heated at 60 °C for 3 hours. The mixture was concentrated under reduced pressure to afford N-formyl-alpha-L-aspartyl-L-phenylalanine dimethyl ester and MDKP in a ratio of 1:2.3.

## Claims

1. 1,4-diacyl-3,6-dioxopiperazine-2(S)-acetic acid compounds, wherein each acyl group, which may be the same or different, is of the formula RC(=O)- wherein R represents hydrogen, a straight chain, branched chain, cyclic or aromatic group containing between 1 and 8 carbon atoms.

2. The compound according to claim 1 which is 1,4-diacetyl-3,6-dioxopiperazine-2(S)-acetic acid.

3. The compound according to claim 1 which is 1,4-diformyl-3,6-dioxopiperazine-2(S)-acetic acid.

4. A process for forming a compound according to anyone of claims 1 to 3 comprising the step of forming a mixture by adding one or more acylating agents to 3,6-dioxopiperazine-2(S)-acetic acid such that two equivalents of acyl groups are provided per equivalent of 3,6-dioxopiperazine-2(S)-acetic acid. `

5. The process according to claim 4 comprising the additional step of heating the 3,6-dioxopiperazine-2(S)-acetic acid acylating agent mixture to between about 25 °C and 130 °C.

6. The process according to claim 4 wherein said adding step takes place in the presence of an inert solvent.

7. 1-acyl-5-benzylidene-3,6-dioxopiperazine-2(S)-acetic acid compounds wherein acyl is a group of the formula RC (=O)- wherein R represents hydrogen, a straight chain, branched chain, cyclic or aromatic group containing between 1 and 8 carbon atoms.

**8.** The compound according to claim 7 which is 1-acetyl-5-benzylidene-3,6-dioxopiperazine-2(S)-acetic acid.

**9.** The compound according to claim 7 which is 1-formyl-5-benzylidene-3,6-dioxopiperazine-2(S)-acetic acid.

**10.** Alkyl 1,4-diacyl-3,6-dioxopiperazine-2(S)-acetate compounds wherein each acyl group, which may be the same or different, is of the formula RC(=O)- wherein R represents hydrogen, a straight chain, branched chain, cyclic or aromatic group containing between 1 and 8 carbon atoms and wherein alkyl comprises a straight chain, or branched chain alkyl group containing between 1 and 7 carbon atoms.

**11.** The compound according to claim 10 which is methyl 1,4-diacetyl-3,6-dioxopiperazine-2(S)-acetate.

**12.** The compound according to claim 10 which is methyl 1,4-diformyl-3,6-dioxopiperazine-2(S)-acetate.

**13.** A process for producing a compound according to anyone of claims 10 to 12 comprising the step of adding one or more acylating agents to an alkyl 3,6-dioxopiperazine-2(S)-acetate compound such that two equivalents of acyl groups are provided per equivalent of alkyl 3,6-dioxopiperazine-2(S)-acetate.

**14.** The process according to claim 13 comprising the additional step of heating said alkyl 3,6-dioxopiperazine-2(S)-acetate acylating agent mixture to between about 25 °C and 130 °C.

**15.** The process according to claim 13 wherein said adding step takes place in an inert solvent.

**16.** Alkyl 1-acyl-5-benzylidene-3,6-dioxopiperazine-2(S)-acetate compounds wherein acyl comprises a group of the formula RC(=O)- wherein R represents hydrogen, a straight chain, branched chain, cyclic or aromatic group having between 1 and 8 carbon atoms, and wherein alkyl comprises a straight chain or branched chain alkyl group containing between 1 and 7 carbon atoms.

**17.** The compound according to claim 16 which is methyl 1-acetyl-5-benzylidene-3,6-dioxopiperazine-2(S)-acetate.

**18.** The compound according to claim 16 which is methyl 1-formyl-5-benzylidene-3,6-dioxopiperazine-2(S)-acetate.

**19.** A process for producing a compound according to any one of claims 16 to 18 comprising the step of adding benzaldehyde in the presence of a base and an inert solvent to an alkyl 1,4-diacyl-3,6-dioxopiperazine-2(S)-acetate compound.

**20.** The process according to claim 19 comprising the additional step of maintaining the reaction medium at a temperature between about -40° C and about 100° C.

**21.** The process according to claim 20, wherein said base comprises an alkoxide compound.

**22.** Alkyl 1-acyl-5(S)-benzyl-3,6-dioxopiperazine-2(S)-acetate compounds, wherein acyl comprises a group of the formula RC(=O)- wherein R represents hydrogen, a straight chain, branched chain, cyclic or aromatic group having between 1 and 8 carbon atoms, and wherein alkyl comprises a straight chain or branched chain alkyl group containing between 1 and 7 carbon atoms.

**23.** The compound according to claim 22 which is methyl 1-acetyl-5(S)-benzyl-3,6-dioxopiperazine-2(S)-acetate.

**24.** The compound according to claim 22 which is methyl 1-formyl-5(S)-benzyl-3,6-dioxopiperazine-2(S)-acetate.

**25.** A process for forming a compound according to any one of claims 22 to 24 comprising the steps of:

(a) adding benzaldehyde to an alkyl 1,4-diacyl-3,6-dioxopiperazine-2(S)-acetate compound in the presence of a base to form an alkyl 1-acyl-5-benzylidene-3,6-dioxopiperazine-2(S)-acetate compound; and
(b) hydrogenating said alkyl 1-acyl-5-benzylidene-3,6-dioxopiperazine-2(S)-acetate compound to form an alkyl 1-acyl-5(S)-benzyl-3,6-dioxopiperazine-2(S)-acetate compound;

wherein each acyl group, which may be the same or different, is of the formula RC(=O)- wherein R represents hydrogen, a straight chain, branched chain, cyclic or aromatic group having between 1 and 8 carbon atoms; and

wherein alkyl comprises a straight chain or branched chain alkyl group containing between 1 and 5 carbon atoms.

**26.** The process according to claim 25, wherein said hydrogenating step comprises contacting said alkyl 1-acetyl-5-benzylidene-3,6-dioxopiperazine-2(S)-acetate compound with gaseous hydrogen or a hydrogen liberating material in the presence of a hydrogenation catalyst selected from the group consisting of Pd, Pt, Ni, Cu, Rh and soluble metal catalysts.

**27.** A process for producing a compound according to anyone of claims 22 to 24 comprising the step of hydrogenating an alkyl 1-acyl-5-benzylidene-3,6-dioxopiperazine-2(S)-acetate compound in an inert solvent.

**28.** The process according to claim 27 wherein said hydrogenation step comprises contacting said alkyl 1-acyl-5-benzylidene-3,6-dioxopiperazine-2(S)-acetate compound with gaseous hydrogen or a hydrogen liberating material in the presence of a hydrogenation catalyst selected from the group consisting of Pd, Pt, Ni, Cu, Rh and soluble metal catalysts.

**29.** 5-benzylidene-3,6-dioxopiperazine-2(S)-acetic acid.

**30.** Alkyl 5-benzylidene-3,6-dioxopiperazine-2(S)-acetate compounds wherein alkyl comprises a straight chain, or branched chain alkyl group containing between 1 and 7 carbon atoms.

**31.** 1-acyl-5-(acyloxybenzyl)-3,6-dioxopiperazine-2(S)-acetic acid compounds wherein acyl is a group of the formula RC(=O)- wherein R represents hydrogen, a straight chain, branched chain, cyclic or aromatic group containing between 1 and 8 carbon atoms.

**32.** The compound according to claim 31 which is 1-acetyl-5-(acetoxybenzyl)-3,6-dioxopiperazine-2(S)-acetic acid.

**33.** The compound according to claim 31 which is N-formyl-5-(formyloxybenzyl)-3,6-dioxopiperazine-2(S)-acetic acid.

**34.** Alkyl 1-acyl-5-(acyloxybenzyl)-3,6-dioxopiperazine-2(S)-acetate compounds, wherein acyl comprises a group of the formula RC(=O)- wherein R represents hydrogen, a straight chain, branched chain, cyclic or aromatic group having between 1 and 8 carbon atoms, and wherein alkyl comprises a straight chain or branched chain alkyl group containing between 1 and 7 carbon atoms.

**35.** The compound according to claim 34 which is methyl 1-acetyl-5-(acetoxybenzyl)-3,6-dioxopiperazine-2(S)-acetate.

**36.** The compound according to claim 34 which is methyl N-formyl-5-(formyloxybenzyl)-3,6-dioxopiperazine-2(S)-acetate.

**37.** A process for producing a compound according to any one of claims 34 to 36 comprising the steps of:

(a) adding benzaldehyde in the presence of a base and an inert solvent to an alkyl 1,4-diacyl-3,6-dioxopiperazine-2(S)-acetate compound;
(b) quenching the compound with acid; and
(c) isolating said produced compound.

**38.** The process according to claim 37 comprising the additional step of:
(d) adding base to the compound produced in step (c) to produce an alkyl 1-acyl-5-benzylidene-3,6-dioxopiperazine-2(S)-acetate compound.

**39.** The process according to claim 37 wherein said base comprises an alkoxide compound.

**40.** The process according to claim 38 wherein said compound produced in step (d) comprises methyl 1-acetyl-5-benzylidene-3,6-dioxopiperazine-2(S)-acetate.

**41.** A process for producing N-acyl-α-L-aspartyl-L-phenylalanine dialkyl ester compounds comprising the steps of:

(a) adding a straght chain, cyclic, aromatic or branched chain alcohol containing between 1 and 7 carbon atoms under basic or neutral conditions to an alkyl 1-acyl-5(S)-benzyl-3,6-dioxopiperazine-2(S)-acetate; and

(b) isolating the N-acyl-α-L-aspartyl-L-phenylalanine dialkyl ester compound produced in step (a);

wherein acyl is a group of the formula RC(=O)- wherein R represents hydrogen, a straight chain, branched chain, cyclic or aromatic group containing between 1 and 8 carbon atoms;
alkyl comprises a straight chain, or branched chain alkyl group containing between 1 and 7 carbon atoms; and dialkyl comprises two alkyl groups which may be the same or different.

**42.** The process according to claim 41 wherein said alcohol comprises methanol, said alkyl 1-acyl-5(S)-benzyl-3,6-dioxopiperazine-2(S)-acetate comprises methyl 1-acetyl-5(S)-benzyl-3,6-dioxopiperazine-2(S)-acetate and wherein said N-acyl-α-L-aspartyl-L-phenylalanine dialkyl ester compound comprises N-acetyl-α-L-aspartyl-L-phenylalanine dimethyl ester.

**43.** The process according to claim 42 wherein step (a) takes place at a temperature between about 20°C and about 60°C for a time period between about 15 minutes and about 24 hours.

**44.** The process according to claim 42 wherein step (a) takes place at a pH ranging from about 7.0 to about 8.5.

**45.** The process according to claim 44 wherein the basic or neutral conditions in step (a) are obtained by adding a base selected from the group consisting of sodium acetate, sodium carbonate, sodium bicarbonate, sodium hydroxide, lithium hydroxide, ammonia, organic amine bases and potassium bases along with said methanol.

**46.** The process according to claim 42 comprising the additional step of:
(c) forming aspartame (α-APM) or a salt thereof by adding HCl, $H_2O$ and $CH_3OH$ to the product obtained in step (b).

**47.** The process according to claim 41, wherein said alcohol comprises methanol, said alkyl 1-acyl-5(S)-benzyl-3,6-dioxopiperazine-2(S)-acetate compound comprises methyl 1-formyl-5(S)-benzyl-3,6-dioxopiperazine-2(S)-acetate and wherein said N-acyl-α-L-aspartyl-L-phenylalanine dialkyl ester compound comprises N-formyl-α-L-aspartyl-L-phenylalanine dimethyl ester.

**48.** A process for producing aspartame (α-APM) or a salt thereof comprising the steps in the order of:

(a) adding one or more acylating agents to an alkyl 3,6-dioxopiperazine-2(S)-acetate compound to form an alkyl 1,4-diacyl-3,6-dioxopiperazine-2(S)-acetate compound;

(b) adding benzaldehyde to said alkyl 1,4-diacyl-3,6-dioxopiperazine-2(S)-acetate compound in the presence of a base to form an alkyl 1-acyl-5-benzylidene-3,6-dioxopiperazine-2(S)-acetate compound;

(c) hydrogenating said alkyl 1-acyl-5-benzylidene-3,6-dioxopiperazine-2(S)-acetate compound to form an alkyl 1-acyl-5(S)-benzyl-3,6-dioxopiperazine-2(S)-acetate compound; and

(d) converting said alkyl 1-acyl-5(S)-benzyl-3,6-dioxopiperazine-2(S)-acetate compound to α-APM or a salt thereof;

wherein each acyl group, which may be the same or different, is of the formula RC(=O)- vherein R represents hydrogen, a straight chain, branched chain, cyclic or aromatic organic group containing between 1 and 8 carbon atoms; and
wherein alkyl comprises a straight chain, or branched chain alkyl group containing between 1 and 7 carbon atoms, or a hydrogen.

**49.** The process according to claim 48 comprising the additional steps of:

(e) converting the residue of the mother liquor produced in step (d) to 5(S)-benzyl-3,6-dioxopiperazine-2(S)-acetic acid or methyl 5(S)-benzyl-3,6-dioxopiperazine-2(S)-acetate; and

(f) partially hydrolyzing said 5(S)-benzyl-3,6-dioxopiperazine-2(S)-acetic acid or methyl 5(S)-benzyl-3,6-dioxopiperazine-2(S)-acetate to $\alpha$-APM or a salt thereof.

**50.** The process according to claim 49 wherein step (e) comprises adjusting the pH of said mother liquor to between about 1.5 and about 6.0 and heating said mother liquor to between about 40°C and about 110°C.

**51.** The process according to claim 49 wherein a cosolvent is added to said mother liquor prior to conversion to 5(S)-benzyl-3,6-dioxopiperazine-2(S)-acetic acid or methyl 5(S)-benzyl-3,6-dioxopiperazine-2(S)-acetate.

**52.** The process according to claim 51 wherein said cosolvent is selected from the group consisting of acetic acid, formic acid and propanoic acid.

**53.** The process according to claim 48 wherein said 1,4-diacyl and 1-acyl substituted compounds comprise 1,4-diacetyl and 1-acetyl substituted compounds respectively and wherein said alkyl esters comprise methyl esters.

**54.** The process according to claim 48 wherein said methyl 3,6-dioxopiperazine-2(S)-acetate is formed by cyclizing

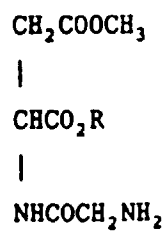

wherein R- H or $CH_3$.

**55.** The process according to claim 54 wherein said

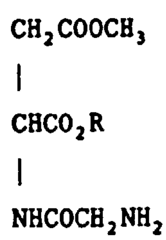

wherein R= H or $CH_3$ is formed by reacting Gly·Asp with methanol in the presence of an acid.

**56.** The process according to claim 55 wherein said Gly·Asp is formed by reacting L-Aspartic Acid with chloroacetylchloride to form chloroacetylaspartic acid and subsequently reacting said chloroacetylaspartic acid with ammonia.

**57.** The process according to claim 53 wherein said acylating agent comprises acetic anhydride.

**58.** The process according to claim 48 wherein said 1,4-diacyl and 1-acyl substituted compounds comprise 1,4-diformyl and 1-formyl substituted compounds respectively and wherein said alkyl esters comprise methyl esters.

**59.** The process according to claim 58 wherein said acylating agent comprises formyl acetic anhydride.

**60.** The process according to claim 48 wherein said hydrogenating step comprises contacting said alkyl 1-acyl-5-benzylidene-3,6-dioxopiperazine-2(S)-acetate compound with gaseous hydrogen or a hydrogen liberating material in the presence of an inert solvent and a hydrogenation catalyst selected from the group consisting of Pd, Pt, Ni, Cu, Rh and soluble metal catalysts.

**61.** The process according to claim 48 wherein said alkyl 1-acyl-5(S)-benzyl-3,6-dioxopiperazine-2(S)-acetate compound comprises a methyl 1-acyl-5(S)-benzyl-3,6-dioxopiperazine-2(S)-acetate compound and wherein step (d) comprises partially hydrolyzing said methyl 1-acyl-5(S)-benzyl-3,6-dioxopiperazine-2(S)-acetate compound to

form α-APM or a salt thereof.

**62.** The process according to claim 48 wherein step (d) comprises:

(i) adding a straight chain, or branched chain alcohol having between 1 and 7 carbon atoms under basic or neutral conditions to said straight chain or branched chain alkyl 1-acyl-5(S)-benzyl-3,6-dioxopiperazine-2(S)-acetate compound to produce a mixture including a N-acyl-α-L-aspartyl-L-phenylalanine dialkyl ester compound; and

(ii) adding HCl, $H_2O$ and $CH_3OH$ to said mixture produced in step (i).

**63.** The process according to claim 62 vhrein step (i) takes place at a temperature between about 20°C and about 60°C for a time period between about 15 minutes and about 24 hours.

**64.** The process according to claim 63 wherein step (i) takes place at a pH ranging from about 7.0 to about 8.5.

**65.** The process according to claim 64 wherein the basic or neutral conditions in step (d) are obtained by adding a base selected from the group consisting of sodium acetate, sodium carbonate, sodium bicarbonate, sodium hydroxide, lithium hydroxide, ammonia, organic amine bases and potassium bases along with said methanol.

**66.** A process for producing Gly·L-Asp comprising the steps of: (a) adding chloroacetylchloride to L-aspartic acid to form a reaction mixture containing chloroacetyl-L-Aspartic acid;

(b) adding ammonia to said chloroacetyl-L-aspartic acid; and

(c) heating the reaction mixture produced in step (b) to between about 50°C and about 250°C for a time period sufficient to form Gly·L-Asp.

**67.** The process according to claim 66 wherein said said time period in step (c) ranges from about 1 second to about 3 hours.

**Patentansprüche**

**1.** 1,4-Diacyl-3,6-dioxopiperazin-2(S)-Essigsäure-Verbindungen, in denen jede Acylgruppe, die gleich oder verschieden sein können, von der Formel RC(=O)- ist, worin R Wasserstoff, eine geradkettige, eine verzweigtkettige, eine zyklische oder eine aromatische Gruppe enthaltend zwischen 1 und 8 Kohlenstoffatome bedeutet.

**2.** Die Verbindung gemäss Anspruch 1, welche 1,4-Diacetyl-3,6-dioxopiperazin-2(S)-Essigsäure ist.

**3.** Die Verbindung gemäss Anspruch 1, welche 1,4-Diformyl-3,6-dioxopiperazin-2(S)-Essigsäure ist.

**4.** Ein Verfahren zur Herstellung einer Verbindung gemäss irgend einem der Ansprüche 1-3 umfassend den Schritt der Bildung einer Mischung durch Zugabe eines oder mehrerer Acylierungsmittel zu 3,6-Dioxopiperazin-2(S)-Essigsäure derart, dass pro Equivalent 3,6-Dioxopiperazin-2(S)-Essigsäure zwei Equivalente Acylgruppen bereitgestellt werden.

**5.** Das Verfahren gemäss Anspruch 4, welches den zusätzlichen Schritt umfasst, dass die Mischung aus 3,6-Dioxopiperazin-2(S)-Essigsäure und Acylierungsmittel auf zwischen etwa 25°C und 130°C erhitzt wird.

**6.** Das Verfahren gemäss Anspruch 4, worin der genannte Zugabeschritt in Anwesenheit eines inerten Lösungsmittels stattfindet.

**7.** 1-Acyl-5-benzyliden-3,6-dioxopiperazin-2(S)-Essigsäure-Verbindungen, in denen Acyl eine Gruppe der Formel RC(=O)- ist, worin R Wasserstoff, eine geradkettige, eine verzweigtkettige, eine zyklische oder eine aromatische Gruppe enthaltend zwischen 1 und 8 Kohlenstoffatome bedeutet.

**8.** Die Verbindung gemäss Anspruch 7, welche 1-Acetyl-5-benzyliden-3,6-dioxopiperazin-2(S)-Essigsäure ist.

9. Die Verbindung gemäss Anspruch 7, welche 1-Formyl-5-benzyliden-3,6-dioxopiperazin-2(S)-Essigsäure ist.

10. Alkyl-1,4-diacyl-3,6-dioxopiperazin-2(S)-acetat-Verbindungen, in denen jede Acylgruppe, welche gleich oder verschieden sein können, die Formel RC(=O)- hat, worin R Wasserstoff, eine geradkettige, eine verzweigtkettige, eine zyklische oder eine aromatische Gruppe enthaltend zwischen 1 und 8 Kohlenstoffatome bedeutet und worin Alkyl eine Alkylgruppe mit gerader oder verzweigter Kette umfasst, welche zwischen 1 und 7 Kohlenstoffatomen enthält.

11. Die Verbindung gemäss Anspruch 10, welche Methyl-1,4-diacetyl-3,6-dioxopiperazin-2(S)-acetat ist.

12. Die Verbindung gemäss Anspruch 10, welche Methyl-1,4-diformyl-3,6-Dioxopiperazin-2(S)-acetat ist.

13. Ein Verfahren für die Herstellung einer Verbindung gemäss irgend einem der Ansprüche 10-12, umfassend den Schritt der Zugabe eines oder mehrerer Acylierungsmittel zu einer Alkyl-3,6-dioxopiperazin-2(S)-acetat-Verbindung, derart, dass pro Equivalent des Alkyl-3,6-dioxopiperazin-2(S)-acetats zwei Equivalente Acylgruppen bereitgestellt werden.

14. Das Verfahren gemäss Anspruch 13, umfassend den zusätzlichen Schritt des Erhitzens der genannten Mischung aus Alkyl-3,6-dioxopiperazin-2(S)-acetat und Acylierungsmittel auf zwischen etwa 25°C und 130°C.

15. Das Verfahren gemäss Anspruch 13, worin der genannte Zugabeschritt in einem inerten Lösungsmittel stattfindet.

16. Alkyl-1-acyl-5-benzyliden-3,6-dioxopiperazin-2(S)-acetat-Verbindungen, in denen Acyl eine Gruppe der Formel RC(=O)- umfasst, worin R Wasserstoff, eine geradkettige, eine verzweigtkettige, eine zyklische oder eine aromatische Gruppe mit zwischen 1 und 8 Kohlenstoffatomen bedeutet und worin Alkyl eine Alkylgruppe mit gerader oder verzweigter Kette umfasst, welche zwischen 1 und 7 Kohlenstoffatomen enthält.

17. Die Verbindung gemäss Anspruch 16, welche Methyl-1-acetyl-5-benzyliden-3,6-dioxopiperazin-2(S)-acetat ist.

18. Die Verbindung gemäss Anspruch 16, welche Methyl-1-formyl-5-benzyliden-3,6-dioxopiperazin-2(S)-acetat ist.

19. Ein Verfahren für die Herstellung einer Verbindung gemäss irgend einem der Ansprüche 16 bis 18 umfassend den Schritt der Zugabe von Benzaldehyd zu einer Alkyl-1,4-diacyl-3,6-dioxopiperazin-2(S)-acetat-Verbindung in Gegenwart einer Base und eines inerten Lösungsmittels.

20. Das Verfahren gemäss Anspruch 19 umfassend den zusätzlichen Schritt das Reaktionsmedium bei einer Temperatur zwischen etwa -40°C und etwa 100°C zu halten.

21. Das Verfahren gemäss Anspruch 20, worin die genannte Base eine Alkoxid-Verbindung umfasst.

22. Alkyl-1-acyl-5(S)-benzyl-3,6-dioxopiperazin-2(S)-acetat-Verbindungen, in denen Acyl eine Gruppe der Formel RC (=O)- umfasst, worin R Wasserstoff, eine geradkettige, eine verzweigtkettige, eine zyklische oder eine aromatische Gruppe mit zwischen 1 und 8 Kohlenstoffatomen bedeutet und worin Alkyl eine Alkylgruppe mit gerader oder verzweigter Kette umfasst, die zwischen 1 und 7 Kohlenstoffatomen enthält.

23. Die Verbindung gemäss Anspruch 22, welche Methyl-1-acetyl-5(S)-benzyl-3,6-dioxopiperazin-2(S)-acetat ist.

24. Die Verbindung gemäss Anspruch 22, welche Methyl-1-formyl-5(S)-benzyl-3,6-dioxopiperazin-2(S)-acetat ist.

25. Ein Verfahren für die Bildung einer Verbindung gemäss irgend einem der Ansprüche 22 bis 24 umfassend die Schritte:

(a) Zugeben von Benzaldehyd zu einer Alkyl-1,4-diacyl-3,6-dioxopiperazin-2(S)-acetat-Verbindung in Anwesenheit einer Base, um eine Alkyl-1-acyl-5-benzyliden-3,6-dioxopiperazin-2(S)-acetat-Verbindung zu bilden; und
(b) Hydrieren der Alkyl-1-acyl-5-benzyliden-3,6-dioxopiperazin-2(S)-acetat-Verbindung, um eine Alkyl-1-acyl-5(S)-benzyl-3,6-dioxopiperazin-2(S)-acetat-Verbindung zu bilden;

worin jede Acylgruppe, welche gleich oder verschieden sein können, die Formel RC(=O)- aufweist, worin R Wasserstoff, eine geradkettige, eine verzweigtkettige, eine zyklische oder eine aromatische Gruppe mit zwischen 1 und 8 Kohlenstoffatomen bedeutet; und

worin Alkyl eine Alkylgruppe mit einer geraden oder verzweigten Kette umfasst, die zwischen 1 und 5 Kohlenstoffatomen enthält.

**26.** Das Verfahren gemäss Anspruch 25, worin der genannte Hydrierungsschritt in Kontakt bringen der Alkyl-1-acetyl-5-benzyliden-3,6-dioxopiperazin-2(S)-acetat-Verbindung mit gasförmigem Wasserstoff oder einem Wasserstoff freisetzenden Material in Gegenwart eines Hydrierkatalysators, ausgewählt aus der Gruppe bestehend aus Pd, Pt, Ni, Cu, Rh und löslichen Metallkatalysatoren, umfasst.

**27.** Ein Verfahren für die Herstellung einer Verbindung gemäss irgend einem der Ansprüche 22 bis 24 umfassend den Schritt des Hydrierens einer Alkyl-1-acyl-5-benzyliden-3,6-dioxopiperazin-2(S)-acetat-Verbindung in einem inerten Lösungsmittel.

**28.** Das Verfahren gemäss Anspruch 27, worin der Hydrierungsschritt in Kontakt bringen der Alkyl-1-acyl-5-benzyliden-3,6-dioxopiperazin-2(S)-acetat-Verbindung mit gasförmigem Wasserstoff oder einem Wasserstoff freisetzenden Material in Anwesenheit eines Hydrierkatalysators ausgewählt aus der Gruppe bestehen aus Pd, Pt, Ni, Cu, Rh und löslichen Metallkatalysatoren, umfasst.

**29.** 5-Benzyliden-3,6-dioxopiperazin-2(S)-Essigsäure.

**30.** Alkyl-5-benzyliden-3,6-dioxopiperazin-2(S)-acetat-Verbindungen, in denen Alkyl eine Alkylgruppe mit gerader oder verzweigter Kette umfasst, die zwischen 1 und 7 Kohlenstoffatomen enthält.

**31.** 1-Acyl-5(acyloxybenzyl)-3,6-dioxopiperazin-2(S)-Essigsäure-Verbindungen, in denen Acyl eine Gruppe der Formel RC(=O)- ist, worin R Wasserstoff, eine geradkettige, eine verzweigtkettige, eine zyklische oder eine aromatische Gruppe enthaltend zwischen 1 und 8 Kohlenstoffatome bedeutet.

**32.** Die Verbindung gemäss Anspruch 31, welche 1-Acetyl-5-(acetoxybenzyl)-3,6-dioxopiperazin-2(S)-Essigsäure ist.

**33.** Die Verbindung gemäss Anspruch 31, welche N-Formyl-5-(formyloxybenzyl)-3,6-dioxopiperazin-2(S)-Essigsäure ist.

**34.** Alkyl-1-acyl-5-(acyloxybenzyl)-3,6-dioxopiperazin-2(S)-acetat-Verbindungen, in denen Acyl eine Gruppe der Formel RC(=O)- umfasst, worin R Wasserstoff, eine geradkettige, eine verzweigtkettige, eine zyklische oder eine aromatische Gruppe mit zwischen 1 und 8 Kohlenstoffatomen bedeutet, und worin Alkyl eine Alkylgruppe mit gerader oder verzweigter Kette umfasst, die zwischen 1 und 7 Kohlenstoffatomen enthält.

**35.** Die Verbindung gemäss Anspruch 34, welche Methyl-1-acetyl-5-(acetoxybenzyl)-3,6-dioxopiperazin-2(S)-acetat ist.

**36.** Die Verbindung gemäss Anspruch 34, welche Methyl-N-formyl-5(formyloxybenzyl)-3,6-dioxopiperazin-2(S)-acetat ist.

**37.** Ein Verfahren für die Herstellung einer Verbindung gemäss irgend einem der Ansprüche 34 bis 36 umfassend die Schritte:

(a) Zugeben von Benzaldehyd zu einer Alkyl-1,4-diacyl-3,6-dioxopiperazin-2(S)-acetat-Verbindung in Anwesenheit einer Base und eines inerten Lösungsmittels;
(b) Quenchen der Verbindung mit Säure; und
(c) Isolieren der hergestellten Verbindung.

**38.** Das Verfahren gemäss Anspruch 37 umfassend den zusätzlichen Schritt:

(d) Zugeben von Base zur in Schritt (c) hergestellten Verbindung, um eine Alkyl-1-acyl-5-benzyliden-3,6-dioxopiperazin-2(S)-acetat-Verbindung herzustellen.

**39.** Das Verfahren gemäss Anspruch 37, worin die Base eine Alkoxidverbindung umfasst.

**40.** Das Verfahren gemäss Anspruch 38, worin die in Schritt (d) hergestellte Verbindung Methyl-1-acetyl-5-benzyliden-3,6-dioxopiperazin-2(S)-acetat umfasst.

**41.** Ein Verfahren für die Herstellung von N-Acyl-α-L-aspartyl-L-phenylalanindialkylester-Verbindungen umfassend die Schritte:

(a) Zugeben eines geradkettigen, zyklischen, aromatischen oder verzweigtkettigen Alkohols enthaltend zwischen 1 und 7 Kohlenstoffatomen unter basischen oder neutralen Bedingungen zu einem Alkyl-1-acyl-5(S)-benzyl-3,6-dioxopiperazin-2(S)-acetat; und
(b) Isolieren der in Schritt (a) hergestellten N-Acyl-α-L-aspartyl-L-phenylalanindialkylester-Verbindung;

wobei Acyl eine Gruppe der Formel RC(=O)- ist, in der R Wasserstoff, eine geradkettige, eine verzweigtkettige, eine zyklische oder eine aromatische Gruppe enthaltend zwischen 1 und 8 Kohlenstoffatomen bedeutet;
Alkyl eine Alkylgruppe mit gerader oder verzweigter Kette umfasst, die zwischen 1 und 7 Kohlenstoff atomen enthält;
Dialkyl zwei Alkylgruppen umfasst, die gleich oder verschieden sein können.

**42.** Das Verfahren gemäss Anspruch 41, worin der Alkohol Methanol umfasst, das Alkyl-1-acyl-5(S)-benzyl-3,6-dioxopiperazin-2(S)-acetat Methyl-1-acetyl-5(S)-benzyl-3,6-dioxopiperazin-2(S)-acetat umfasst und worin die N-Acyl-α-L-aspartyl-L-phenylalanindialkylester-Verbindung N-Acetyl-α-L-aspartyl-L-phenylalanindimethylester umfasst.

**43.** Das Verfahren gemäss Anspruch 42, worin Schritt (a) bei einer Temperatur zwischen etwa 20°C und etwa 60°C während eines Zeitraums zwischen etwa 15 Minuten und etwa 24 Stunden stattfindet.

**44.** Das Verfahren gemäss Anspruch 42, worin Schritt (a) bei einem pH im Bereich von etwa 7,0 bis etwa 8,5 stattfindet.

**45.** Das Verfahren gemäss Anspruch 44, worin die basischen oder neutralen Bedingungen in Schritt (a) durch Zugabe einer Base ausgewählt aus der Gruppe, bestehend aus Natriumacetat, Natriumcarbonat, Natriumbicarbonat, Natriumhydroxid, Lithiumhydroxid, Ammoniak, organischen Aminbasen und Kaliumbasen zusammen mit dem Methanol erhalten werden.

**46.** Das Verfahren gemäss Anspruch 42, umfassend den zusätzlichen Schritt:
(c) Bilden von Aspartam (α-APM) oder einem Salz desselben durch Zugabe von HCl, $H_2O$ und $CH_3OH$ zum in Schritt (b) erhaltenen Produkt.

**47.** Das Verfahren gemäss Anspruch 41, worin der genannte Alkohol Methanol umfasst, die genannte Alkyl-1-acyl-5(S)-benzyl-3,6-dioxopiperazin-2(S)-acetat-Verbindung Methyl-1-formyl-5(S)-benzyl-3,6-dioxopiperazin-2(S)-acetat umfasst und worin die genannte N-Acyl-α-L-aspartyl-L-phenylalanindialkylester-Verbindung N-Formyl-α-L-aspartyl-L-phenylalanindimethylester umfasst.

**48.** Ein Verfahren für die Herstellung von Aspartam (α-APM) oder eines Salzes desselben umfassend die Schritte in der Reihenfolge:

(a) Zugeben eines oder mehrerer Acylierungsmittel zu einer Alkyl-3,6-dioxopiperazin-2(S)-acetat-Verbindung, um eine Alkyl-1,4-diacyl-3,6-dioxopiperazin-2(S)-acetat-Verbindung zu bilden;
(b) Zugeben von Benzaldehyd zur Alkyl-1,4-diacyl-3,6-dioxopiperazin-2(S)-acetat-Verbindung in Anwesenheit einer Base, um eine Alkyl-1-acyl-5-benzyliden-3,6-dioxopiperazin-2(S)-acetat-Verbindung zu bilden;
(c) Hydrieren der Alkyl-1-acyl-5-benzyliden-3,6-dioxopiperazin-2(S)-acetat-Verbindung, um eine Alkyl-1-acyl-5(S)-benzyl-3,6-dioxopiperazin-2(S)-Verbindung zu bilden; und
(d) Ueberführen der Alkyl-1-acyl-5(S)-benzyl-3,6-dioxopiperazin-2(S)-acetat-Verbindung in α-APM oder ein Salz desselben;

wobei jede Acylgruppe, die gleich oder verschieden sein können, die Formel RC(=O)- hat, in der R Wasserstoff, eine geradkettige, eine verzweigtkettige, eine zyklische oder eine aromatische organische Gruppe enthaltend zwischen 1 und 8 Kohlenstoffatomen bedeuten; und
worin Alkyl eine geradkettige oder verzweigtkettige Alkylgruppe, enthaltend zwischen 1 und 7 Kohlenstoffatomen, oder ein Wasserstoff umfasst.

**49.** Das Verfahren gemäss Anspruch 48 umfassend die zusätzlichen Schritte:

(e) Ueberführen des Rests der Mutterlauge, die in Schritt (d) hergestellt wurde, in 5(S)-Benzyl-3,6-dioxopiperazin-2(S)-Essigsäure oder Methyl-5(S)-benzyl-3,6-dioxopiperazin-2(S)-acetat; und
(f) partielles Hydrolysieren der 5(S)-Benzyl-3,6-dioxopiperazin-2(S)-Essigsäure oder des Methyl-5(S)-benzyl-3,6-dioxopiperazin-2(S)-acetats zu α-APM oder einem Salz desselben.

**50.** Das Verfahren gemäss Anspruch 49, worin Schritt (e) die Einstellung des pH der Mutterlauge auf zwischen etwa 1,5 und etwa 6,0 und Erhitzen der Mutterlauge auf zwischen etwa 40°C und etwa 110°C umfasst.

**51.** Das Verfahren gemäss Anspruch 49, in dem vor der Umsetzung zu 5(S)-Benzyl-3,6-dioxopiperazin-2(S)-Essigsäure oder Methyl-5(S)-benzyl-3,6-dioxopiperazin-2(S)-acetat der Mutterlauge ein Cosolvens zugegeben wird.

**52.** Das Verfahren gemäss Anspruch 51, in dem das Cosolvens ausgewählt ist aus der Gruppe bestehend aus Essigsäure, Ameisensäure und Propionsäure.

**53.** Das Verfahren gemäss Anspruch 48, in dem die 1,4-Diacyl- und 1-Acyl-substituierten Verbindungen 1,4-Diacetyl- resp. 1-Acetyl-substituierte Verbindungen umfassen und worin die Alkylester Methylester umfassen.

**54.** Das Verfahren gemäss Anspruch 48, worin das Methyl-3,6-dioxopiperazin-2(S)-acetat durch Zyklisieren von

$$\begin{array}{l} CH_2COOCH_3 \\ | \\ CHCO_2R \\ | \\ NHCOCH_2NH_2 \end{array} ,$$

worin R= H oder $CH_3$ bedeutet, gebildet wird.

**55.** Das Verfahren gemäss Anspruch 54, worin das

$$\begin{array}{l} CH_2COOCH_3 \\ | \\ CHCO_2R \\ | \\ NHCOCH_2NH_2 \end{array} ,$$

in dem R = H oder $CH_3$ ist, durch zur Reaktion bringen von Gly·Asp mit Methanol in Anwesenheit einer Säure gebildet wird.

**56.** Das Verfahren gemäss Anspruch 55, worin das Gly·Asp gebildet wird durch zur Reaktion bringen von L-Asparaginsäure mit Chloracetylchlorid um Chloracetylasparaginsäure zu bilden und nachfolgendes zur Reaktion bringen der Chloracetylasparaginsäure mit Ammoniak.

**57.** Das Verfahren gemäss Anspruch 53, worin das Acylierungsmittel Essigsäureanhydrid umfasst.

**58.** Das Verfahren gemäss Anspruch 48, worin die 1,4-Diacyl- und 1-Acyl-substituierten Verbindungen 1,4-Diformyl- resp. 1-Formyl-substituierte Verbindungen umfassen und worin die genannten Alkylester Methylester umfassen.

**59.** Das Verfahren gemäss Anspruch 58, worin das Acylierungsmittel Ameisensäure-Essigsäure-Anhydrid umfasst.

**60.** Das Verfahren gemäss Anspruch 48, worin der Hydrierungsschritt in Kontakt bringen der Alkyl-1-acyl-5-benzyliden-3,6-dioxopiperazin-2(S)-acetat-Verbindung mit gasförmigem Wasserstoff oder einem Wasserstoff freisetzenden Material in Anwesenheit eines inerten Lösungsmittels und eines Hydrierkatalysators, ausgewählt aus der Gruppe

bestehend aus Pd, Pt, Ni, Cu, Rh und löslichen Metallkatalysatoren, umfasst.

**61.** Das Verfahren gemäss Anspruch 48, worin die Alkyl-1-acyl-5(S)-benzyl-3,6-dioxopiperazin-2(S)-acetat-Verbindung eine Methyl-1-acyl-5(S)-benzyl-3,6-dioxopiperazin-2(S)-acetat-Verbindung umfasst und worin Schritt (d) das teilweise Hydrolysieren der Methyl-1-acyl-5(S)-benzyl-3,6-dioxopiperazin-2(S)-acetat-Verbindung umfasst, um α-APM oder ein Salz desselben zu bilden.

**62.** Das Verfahren gemäss Anspruch 48, worin Schritt (d) umfasst:

(i) Zugeben eines geradkettigen oder verzweigtkettigen Alkohols mit zwischen 1 und 7 Kohlenstoffatomen unter basischen oder neutralen Bedingungen zur geradkettigen oder verzweigtkettigen Alkyl-1-acyl-5(S)-benzyl-3,6-dioxopiperazin-2(S)-acetat-Verbindung um eine Mischung herzustellen, die eine N-Acyl-α-L-aspartyl-L-phenylalanindialkylester-Verbindung einschliesst; und
(ii) Zugeben von HCl, $H_2O$ und $CH_3OH$ zur in Schritt (i) hergestellten Mischung.

**63.** Das Verfahren gemäss Anspruch 62, worin Schritt (i) bei einer Temperatur zwischen etwa 20°C und etwa 60°C während eines Zeitraums zwischen etwa 15 Minuten und etwa 24 Stunden stattfindet.

**64.** Das Verfahren gemäss Anspruch 63, worin Schritt (i) bei einem pH im Bereich von etwa 7,0 bis etwa 8,5 stattfindet.

**65.** Das Verfahren gemäss Anspruch 64, worin die basischen oder neutralen Bedingungen in Schritt (d) durch Zugeben einer Base, ausgewählt aus der Gruppe bestehend aus Natriumacetat, Natriumcarbonat, Natriumbicarbonat, Natriumhydroxid, Lithiumhydroxid, Ammoniak, organischen Aminbasen und Kaliumbasen, zusammen mit dem Methanol erhalten werden.

**66.** Ein Verfahren für die Herstellung von Gly·L-Asp umfassend die Schritte:

(a) Zugeben von Chloracetylchlorid zu L-Asparaginsäure um eine Reaktionsmischung enthaltend Chloracetyl-L-asparaginsäure zu bilden;
(b) Zugeben von Ammoniak zur Chloracetyl-L-asparaginsäure; und
(c) Erhitzen der in Schritt (b) hergestellten Reaktionsmischung auf zwischen etwa 50°C und etwa 250°C während eines Zeitraums, der ausreichend ist um Gly·L-Asp zu bilden.

**67.** Das Verfahren gemäss Anspruch 66, worin der genannte Zeitraum in Schritt (c) von etwa 1 Sekunde bis etwa 3 Stunden beträgt.

**Revendications**

**1.** Des composés de 1,4-diacyle-3,6-dioxopipérazine-2(S)-acide acétique, dans lesquels chaque groupe d'acyle, qui peut être le même ou différent, est de la formule RC(=O)- dont R représente l'hydrogène, un groupe d'une chaîne droite, d'une chaîne ramifiée, cyclique ou aromatique contenant entre 1 à 8 atomes de carbone.

**2.** Le composé suivant la revendication 1 qui est le 1,4-diacétyle-3,6-dioxopipérazine-2(S)-acide acétique.

**3.** Le composé suivant la revendication 1 qui est le 1,4-diformyle-3,6-dioxopipérazine-2(S)-acide acétique.

**4.** Un procédé pour former un composé suivant une quelconque des revendications 1 à 3, comprenant l'étape de formation d'un mélange en rajoutant un ou plusieurs agents d'acylation au 3,6-dioxopipérazine-2(S)-acide acétique, d'une manière que deux équivalents de groupes d'acyle soient fournis par équivalent de 3,6-dioxopipérazine-2(S)-acide acétique.

**5.** Le procédé suivant la revendication 4 comprenant l'étape supplémentaire de chauffage du mélange de 3,6-dioxopipérazine-2(S)-acide acétique et de l'agent d'acylation à environ 25°C et 130°C.

**6.** Le procédé suivant la revendication 4 dont ladite étape d'addition a lieu en présence d'un solvant inerte.

**7.** Des composés de 1-acyle-5-benzylidène-3,6-dioxopipérazine-2(S)-acide acétiques dont l'acyle est un groupe de

la formule RC(=O)- dont R représente l'hydrogène, un groupe d'une chaîne droite, d'une chaîne ramifiée, cyclique ou aromatique contenant entre 1 à 8 atomes de carbone.

**8.** Le composé suivant la revendication 7 qui est le 1-acétyle-5-benzylidène-3,6-dioxopipérazine-2(S)-acide acétique.

**9.** Le composé suivant la revendication 7 qui est le l-formyle-5-benzylidène-3,6-dioxopipérazine-2(S)-acide acétique.

**10.** Des composés d'alkyle 1,4-diacyle-3,6-dioxopipérazine-2(S)-acétate dont chaque groupe d'acyle, qui peut être le même ou différent, est un groupe de la formule RC(=O)- dont R représente l'hydrogène, un groupe d'une chaîne droite, d'une chaîne ramifiée, cyclique ou aromatique contenant entre 1 à 8 atomes de carbone et dont l'alkyle comprend un groupe d'une chaîne droite, d'une chaîne ramifiée contenant entre 1 et 7 atomes de carbone.

**11.** Le composé suivant la revendication 10 qui est le méthyle 1,4-diacétyle-3,6-dioxopipérazine-2(S)-acétate.

**12.** Le composé suivant la revendication 10 qui est le méthyle 1,4-diformyle-3,6-dioxopipérazine-2(S)-acétate.

**13.** Un procédé pour préparer un composé suivant une quelconque des revendications 10 à 12, comprenant l'étape d'addition d'un ou plusieurs agents d'acylation à un composé d'alkyle 3,6-dioxopipérazine-2(S)-acétate d'une manière que deux équivalents de groupes d'acyle soient fournis par équivalent d'alkyle 3,6-dioxopipérazine-2(S)-acétate.

**14.** Le procédé suivant la revendication 13 comprenant l'étape supplémentaire de chauffer ledit mélange d'alkyle 3,6-dioxopipérazine-2(S)-acétate et d'agent d'acylation entre environ 25°C et 130°C.

**15.** Le procédé suivant la revendication 13 dont ladite étape d'addition a lieu en présence d'un solvant inerte.

**16.** Les composés d'alkyle 1-acyle-5-benzylidène-3,6-dioxopipérazine-2(S)-acétate dont l'acyle est un groupe de la formule RC(=O)- dont R représente l'hydrogène, un groupe d'une chaîne droite, d'une chaîne ramifiée, cyclique ou aromatique contenant entre 1 à 8 atomes de carbone et dont l'alkyle comprend un groupe d'une chaîne droite, d'une chaîne ramifiée contenant 1 et 7 atomes de carbone.

**17.** Le composé suivant la revendication 16 qui est le méthyle 1-acétyle-5-benzylidène-3,6-dioxopipérazine-2(S)-acétate.

**18.** Le composé suivant la revendication 16 qui est le méthyle 1-formyle-5-benzylidène-3,6-dioxopipérazine-2(S)-acétate.

**19.** Un procédé pour la fabrication d'un composé suivant une quelconque des revendications 16 à 18 comprenant l'étape de l'addition du benzaldéhyde en présence d'une base et d'un solvant inerte à un composé d'alkyle 1,4-diacyle-3,6-dioxopipérazine-2(S)-acide acétique.

**20.** Un procédé suivant la revendication 19 comprenant l'étape supplémentaire de maintenir le milieu réactionnel à une température entre environ -40°C et environ 100°C.

**21.** Un procédé suivant la revendication 20, dont ladite base comprend un composé d'alkoxide.

**22.** Des composés d'alkyle 1-acyle-5(S)-benzyl-3,6-dioxopipérazine-2(S)-acétate dont l'acyle comprend un groupe de la formule RC(=O)- dont R représente l'hydrogène, un groupe d'une chaîne droite, d'une chaîne ramifiée, un groupe cyclique ou aromatique contenant entre 1 à 8 atomes de carbone et dont l'alkyle comprend un groupe d'alkyle d'une chaîne droite, d'une chaîne ramifiée contenant 1 et 7 atomes de carbone.

**23.** Un composé suivant la revendication 22 qui est le méthyle 1-acétyle-5(S)-benzyle-3,6-dioxopipérazine-2(S)-acétate.

**24.** Un composé suivant la revendication 22 qui est le méthyle 1-formyle-5(S)-benzyle-3,6-dioxopipérazine-2(S)-acétate.

**25.** Un procédé pour la formation d'un composé suivant une quelconque des revendications 22 à 24 compre-nant les

étapes de:

(a) l'addition du benzaldéhyde à un composé d'alkyle 1,4-diacyle-3,6-dioxopipérazine-2(S)-acétate en présence d'une base afin de former un composé d'alkyle 1-acyle-5-benzylidène-3,6-dioxopipérazine-2(S)-acétate; et
(b) l'hydrogénation dudit composé d'alkyle 1-acyle-5-benzylidène-3,6-dioxopipérazine-2(S)-acétate afin de former le composé d'alkyle 1-acyle-5-(S)-benzyle-3,6-dioxopipérazine-2(S)-acétate;

dont chaque groupe d'acyle qui peut être le même ou être différent, est de la formule RC(=O)- dont R représente l'hydrogène, un groupe d'une chaîne droite, d'une chaîne ramifiée, un groupe cyclique ou aromatique contenant entre 1 à 8 atomes de carbone; et

dont l'alkyle comprend un groupe d'alkyle d'une chaîne droite, d'une chaîne ramifiée contenant entre 1 et 5 atomes de carbone.

**26.** Le procédé suivant la revendication 25, dont ladite étape d'hydrogénation comprend la mise en contact dudit composé 1-acétyle-5-benzylidène-3,6-dioxopipérazine-2(S)-acétate avec de l'hydrogène gazeux ou avec un matériel libérant de l'hydrogène, en présence d'un produit catalysant l'hydrogénation, sélectionné du groupe comprenant le Pd, Pt, Ni, Cu, Rh et des produits catalytiques solubles de métal.

**27.** Un procédé pour la production d'un composé suivant une quelconque des revendications 22 à 24 comprenant l'étape d'hydrogénation d'un composé d'alkyle 1-acyle-5-benzylidène-3,6-dioxopipérazine-2(S)-acétate dans un solvant inerte.

**28.** Le procédé suivant la revendication 27, dont ladite étape d'hydrogénation comprend la mise en contact dudit composé d'alkyle 1-acyle-5-benzylidène-3,6-dioxopipérazine-2(S)-acétate avec de l'hydrogène gazeux ou en présence d'un produit catalysant l'hydrogénation, sélectionné du groupe compre-nant le Pd, Pt, Ni, Cu, Rh et des produits catalytiques solubles de métal.

**29.** Le 5-benzylidène-3,6-dioxopipérazine-2(S)-acide acétique.

**30.** Des composés d'alkyle 5-benzylidène-3,6-dioxopipérazine-2(S)-acétate, dont l'alkyle comprend un groupe d'alkyle d'une chaîne droite, d'une chaîne ramifiée contenant entre 1 à 7 atomes de carbone.

**31.** Des composés 1-acyle-5-(acyloxybenzyle)-3,6-dioxopipérazine-2(S)-acide acétiques dont l'acyle est un groupe de la formule RC(=O)-, dont R représente l'hydrogène, un groupe d'alkyle d'une chaîne droite, d'une chaîne ramifiée, un groupe cyclique ou aromatique contenant entre 1 à 8 atomes de carbone.

**32.** Le composé suivant la revendication 31 qui est le 1-acétyle-5-(acétoxybenzyle)-3,6-dioxopipérazine-2(S)-acide acétique.

**33.** Le composé suivant la revendication 31 qui est le N-formyle-5-(formyloxybenzyle)-3,6-dioxopipérazine-2(S)-acide acétique.

**34.** Des composés d'alkyle 1-acyle-5-(acyloxybenzyle)-3,6-dioxopipérazine-2(S)-acétate, dont l'acyle comprend un groupe de la formule RC(=O)-, dont R représente l'hydrogène, un groupe d'une chaîne droite, d'une chaîne ramifiée, un groupe cyclique ou aromatique contenant entre 1 à 8 atomes de carbone et dont l'alkyle comprend un groupe d'alkyle d'une chaîne droite, d'une chaîne ramifiée contenant entre 1 à 7 atomes de carbone.

**35.** Le composé suivant la revendication 34 qui est le méthyle 1-acétyle-5-(acétoxybenzyle)-3,6-dioxopipérazine-2(S)-acétate.

**36.** Le composé suivant la revendication 34 qui est le méthyle 1-formyle-5-(formyloxybenzyle)-3,6-dioxopipérazine-2(S)-acétate.

**37.** Un procédé pour la fabrication d'un composé suivant l'une des revendications 34 à 36 compre-nant les étapes de:

(a) l'addition du benzaldéhyde à un composé d'alkyle 1,4-diacyle-3,6-dioxopipérazine-2(S)-acide acétique en présence d'une base et un solvant inerte;

(b) l'arrêt du composé avec de l'acide; et
(c) l'isolation dudit composé fabriqué.

**38.** Le procédé suivant la revendication 37 comprenant l'étape supplémentaire de:
(d) l'addition d'une base au composé préparé dans l'étape (c) afin de préparer un composé d'alkyle 1-acyle-5-benzylidène-3,6-dioxopipérazine-2(S)-acétate.

**39.** Le procédé suivant la revendication 37 dont ladite base comprend un composé d'alkoxyde.

**40.** Le procédé suivant la revendication 38 dont ledit composé préparé dans l'étape (d) comprend le méthyle 1-acétyle-5-benzylidène-3,6-dioxopipérazine-2(S)-acétate.

**41.** Un procédé pour la fabrication des composés de l'ester dialkylique du N-acyle-α-L-aspartyle-L-phénylalanine comprenant les étapes de

(a) l'addition d'un alcool d'une chaîne droite, cyclique, aromatique ou d'une chaîne ramifiée contenant entre 1 à 7 atomes de carbone sous des conditions basiques ou neutres à un alkyle 1-acyle-5(S)-benzyle-3,6-dioxopipérazine-2(S)-acétate; et
(b) l'isolation du composé de diester dialkylique du N-acyle-α-L-aspartyle-L-phénylalanine préparé dans l'étape (a);

dont l'acyle est de la formule RC(=O)-, dont R représente l'hydrogène, un groupe d'alkyle d'une chaîne droite, d'une chaîne ramifiée, un groupe cyclique ou aromatique contenant entre 1 à 8 atomes de carbone;
l'alkyle comprend un groupe d'alkyle d'une chaîne droite, d'une chaîne ramifiée contenant entre 1 à 7 atomes de carbone; et
le dialkyle comprend deux groupes d'alkyle qui peuvent être les mêmes ou différents.

**42.** Le procédé suivant la revendication 41 dont ledit alcool comprend le méthanol, ledit alkyle 1-acyle-5(S)-benzyle-3,6-dioxopipérazine-2(S)-acétate comprend le méthyle 1-acétyle-5(S)-benzyle-3,6-dioxopipérazine-2(S)-acétate et dont ledit ester dialkylique du N-acyle-α-L-aspartyle-L-phénylalanine comprend l'ester dialkylique du N-acétyle-α-L-aspartyle-L-phénylalanine.

**43.** Le procédé suivant la revendication 42 dont l'étape (a) a lieu à une température comprise entre environ 20°C et environ 60°C pendant une période comprise entre environ 15 minutes et environ 24 heures.

**44.** Le procédé suivant la revendication 42 dont l'étape (a) a lieu à un pH qui varie entre environ 7,0 et environ 8,5.

**45.** Le procédé suivant la revendication 44 dont les conditions basiques ou neutres dans l'étape (a) sont obtenues par l'addition d'une base sélectionnée du groupe consistant en de l'acétate de sodium, du carbonate de sodium, du bicarbonate de sodium, de l'hydroxide de sodium, de l'hydroxyde de lithium, de l'ammoniaque, des bases organiques et des bases de potassium ensembles avec ledit méthanole.

**46.** Le procédé suivant la revendication 42 comprenant l'étape supplémentaire de:
(c) la formation de l'aspartame (α-APM) ou un sel de ce-dernier en rajoutant du HC, du $H_2O$ et du $CH_3OH$ dans le produit obtenu dans l'étape (b).

**47.** Le procédé suivant la revendication 41, dont ledit alcool comprend le méthanol, ledit composé d'alkyle 1-acyle-5(S)-benzyle-3,6-dioxopipérazine-2(S)-acétate comprend le méthyle 1-formyle-5(S)-benzyle-3,6-dioxopipérazine-2(S)-acétate et dont ledit ester dialkylique du N-acyle-α-L-aspartyle-L-phénylalanine comprend l'ester diméthylique du N-formyle-α-L-aspartyle-L-phénylalanine.

**48.** Un procédé pour la fabrication de l'aspartame (α-APM) ou un sel de ce dernier comprenant les étapes dans l'ordre de:

(a) l'addition d'un ou plusieurs agents d'acylation à un composé d'alkyle 3,6-dioxopipérazine-2(S)-acétate afin de former un composé d'alkyle 1,4-diacyle-3,6-dioxopipérazine-2(S)-acétate;

(b) l'addition du benzaldéhyde au composé d'alkyle 1,4-diacyle-3,6-dioxopipérazine-2(S)-acétate en présence

d'une base afin de former un composé d'alkyle 1-acyle-5-benzylidène-3,6-dioxopipérazine-2(S)-acétate;

(c) l'hydrogénation dudit composé d'alkyle 1-acyle-5-benzylidène-3,6-dioxopipérazine-2(S)-acétate afin de former un composé d'alkyle 1-acyle-5(S)-benzyle-3,6-dioxopipérazine-2(S)-acétate; et

(d) la conversion dudit composé d'alkyle 1-acyle-5(S)-benzyle-3,6-dioxopipérazine-2(S)-acétate à l'α-APM ou un sel de ce-dernier;

dont chaque groupe d'acyle, qui peut être le même ou différent, est de la formule RC(=O)- dont R représente l'hydrogène, un groupe d'alkyle d'une chaîne droite, d'une chaîne ramifiée, cyclique ou aromatique contenant entre 1 à 8 atomes de carbone; et

dont l'alkyle comprend un groupe d'alkyle de chaîne droite, d'une chaîne ramifiée contenant 1 et 7 atomes de carbone ou d'un hydrogène.

**49.** Le procédé suivant la revendication 48 comprenant les étapes supplémentaires de:

(e) la conversion du résidu du liquide d'origine préparé dans l'étape (d) au 5(S)-benzyle-3,6-dioxopipérazine-2(S)-acide acétique ou au méthyle 5(S)-benzyle-3,6-dioxopipérazine-2(S)-acétate; et

(f) l'hydrolysation partielle dudit 5(S)-benzyle-3,6-dioxopipérazine-2(S)-acide acétique ou du méthyle 5(S)-benzyle-3,6-dioxopipérazine-2(S)-acétate à l'α-APM ou un sel de ce-dernier.

**50.** Le procédé suivant la revendication 49 dont l'étape (e) comprend l'ajustement du pH dudit liquide mère entre environ 1,5 et environ 6,0 et le chauffage dudit liquide mère entre environ 40°C et environ 110°C.

**51.** Le procédé suivant la revendication 49 dont un co-solvant est rajouté au liquide original avant la conversion au 5 (S)-benzyle-3,6-dioxopipérazine-2(S)-acide acétique ou du méthyle 5(S)-benzyle-3,6-dioxopipérazine-2(S)-acétate.

**52.** Le procédé suivant la revendication 51 dont ledit co-solvant est sélectionné du groupe consistant en de l'acide acétique, de l'acide formique et de l'acide propanoïc.

**53.** Le procédé suivant la revendication 48 dont lesdits composés substitués de 1,4-diacyle et 1-acyle comprennent les composés substitués de 1,4-diacétyle et le 1-acétyle et dont lesdits esters d'alkyle comprennent les esters de méthyle.

**54.** Le procédé suivant la revendication 48 dont ledit méthyle 3,6-dioxopipérazine-2(S)-acétate est formé en soumettant à une cyclisation le

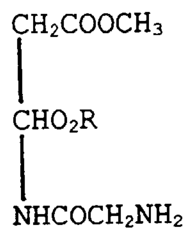

dont R = H ou $CH_3$.

**55.** Le procédé suivant la revendication 54 dans lequel ledit

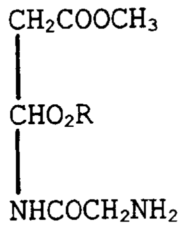

dont R = H ou $CH_3$ est formé en faisant réagir le Gly·Asp avec du méthanol en présence d'un acide.

**56.** Le procédé suivant la revendication 55 dont ledit Gly·Asp est formé en faisant réagir de l'acide L-aspartique avec du chlorure de chloracétyle afin de former de l'acide chloracétylaspartique et ensuite faire réagir ledit acide chloracétylaspartique avec de l'ammoniaque.

**57.** Le procédé suivant la revendication 53 dont ledit agent d'acylation comprend l'anhydride acétique.

**58.** Le procédé suivant la revendication 48 dont lesdits composés substitués de 1,4-diacyle et 1-acyle comprennent les composés substitués de 1,4-diformyle et 1-formyle, respectivement, et dont lesdits esters d'alkyle comprennent des esters de méthyle.

**59.** Le procédé suivant la revendication 58 dont lesdits agents d'acylation comprennent l'anhydride formyle acétique.

**60.** Le procédé suivant la revendication 48 dont ladite étape d'hydrogénation comprend la mise en contact dudit composé d'alkyle l-acyle-5(S)-benzylidène-3,6-dioxopipérazine-2(S)-acétate avec de l'hydrogène gazeux ou du matériel libérant de l'hydrogène en présence d'un solvant inerte et un produit catalytique d'hydrogénation sélectionné du groupe consistant de Pd, Pt, Ni, Cu, Rh et des produits catalytiques solubles de métal.

**61.** Le procédé suivant la revendication 48 dont ledit composé d'alkyle 1-acyle-5(S)-benzyle-3,6-dioxopipérazine-2(S)-acétate comprend un composé de méthyle 1-acyle-5(S)-benzyle-3,6-dioxopipérazine-2(S)-acétate et dont l'étape (d) comprend l'hydrolyse partielle dudit composé de méthyle 1-acyle-5(S)-benzyle-3,6-dioxopipérazine-2(S)-acétate afin de former d'α-APM ou un sel de ce-dernier.

**62.** Le procédé suivant la revendication 48 dont ladite étape (d) comprend:

(i) l'addition d'un alcool d'une chaine droite ou ramifiée avec entre 1 à 7 atomes de carbone sous des conditions basiques ou neutres au dit composé d'alkyle 1-acyle-5(S)-benzyle-3,6-dioxopipérazine-2(S)-acétate d'une chaine droite ou ramifiée afin de préparer un mélange incluant un composé d'ester dialkylique du N-acyle-α-L-aspartyle-L-phénylalanine; et

(ii) l'addition de HCl, $H_2O$, et $CH_3OH$ au dit mélange préparé dans l'étape (i).

**63.** Le procédé suivant la revendication 62 dont ladite étape (i) a lieu à une température comprise entre environ 20°C et environ 60°C pendant une période d'entre 15 minutes et environ 24 heures.

**64.** Le procédé suivant la revendication 63 dont ladite étape (i) a lieu à un pH qui varie entre environ 7,0 et environ 8,5.

**65.** Le procédé suivant la revendication 64 dont les conditions neutres ou basiques dans l'étape (d) sont obtenues en rajoutant une base sélectionnée du groupe consistant de l'acétate de sodium, du carbonate de sodium, du bicarbonate de sodium, de l'hydroxyde de sodium, de l'hydroxide de lithium, de l'ammoniaque, des bases d'amines organiques et des bases de potassium ensembles avec ledit méthanol.

**66.** Un procédé pour fabriquer du Gly·L-Asp comprenant les étapes suivantes:

(a) l'addition du chlorure de chloracétyle à l'acide L-aspartique afin de former un mélange réactionnel contenant de l'acide chloracétyl-L-aspartique;
(b) l'addition de l'ammoniaque au dit acide chloracétyl-L-aspartique; et
(c) le chauffage du mélange réactionnel fabriqué dans l'étape (b) à entre 50°C et environ 250°C pendant une

période suffisante pour former du Gly·L-Asp.

**67.** Le procédé suivant la revendication 66 dont ladite période dans l'étape (c) varie entre environ 1 seconde à environ 3 heures.